(19) Europäisches Patentamt / European Patent Office / Office européen des brevets

(11) **EP 3 181 152 A1**

(12) **EUROPEAN PATENT APPLICATION**

(43) Date of publication:
21.06.2017 Bulletin 2017/25

(21) Application number: 15201164.9

(22) Date of filing: 18.12.2015

(51) Int Cl.:
*A61L 15/24* (2006.01)     *A61L 15/32* (2006.01)
*A61L 15/58* (2006.01)     *C08L 1/26* (2006.01)
*C07K 14/78* (2006.01)     *A61L 15/26* (2006.01)
*C08L 71/02* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: BSN medical GmbH
20253 Hamburg (DE)

(72) Inventors:
• Lanfer, Babette
20149 Hamburg (DE)

• Arshi, Annahit
20357 Hamburg (DE)
• Hemmrich, Karsten
40589 Düsseldorf (DE)
• Wilhelms, Tim
22041 Hamburg (DE)
• Blumenthal, Nils
22765 Hamburg (DE)

(74) Representative: Jostarndt Patentanwalts-AG
Philipsstrasse 8
52068 Aachen (DE)

(54) **MULTI-LAYERED WOUND CARE PRODUCT**

(57) The invention relates to a multilayered wound care product comprising a nanocellulose layer and a hydrogel layer comprising at least one ECM-mimicking structure. The invention further relates to the use of said wound care product for plastic surgery, tissue engineering and for treatment of acute and chronic wounds. The invention finally relates to a method of producing said multi-layered wound care product.

## Description

### Field of the invention:

The invention relates to a multilayered wound care product comprising a nanocellulose layer and a hydrogel layer comprising at least one ECM-mimicking structure. The invention further relates to the use of said wound care product for plastic surgery, tissue engineering and for treatment of acute and chronic wounds. The invention finally relates to a method of producing said multi-layered wound care product.

### Background of the invention

Wound healing is a dynamic process involving interactions between cells, extracellular matrix (ECM) and growth factors that reconstitute tissue following injury. The extracellular matrix (ECM) plays an important role in tissue regeneration and is the major component of the dermal skin layer. The composition of ECM includes proteoglycans, hyaluronic acid, collagen, fibronectin and elastin. As well as providing a structural support for cells, some components of the ECM bind to growth factors, creating a reservoir of active molecules that can be rapidly mobilized following injury to stimulate cell proliferation and migration. In many chronic wounds, increased levels of inflammatory cells lead to elevated levels of proteases that appear to degrade the ECM components, growth factors, protein and receptors that are essential for healing.

Recognition of the importance of the ECM in wound healing has led to the development of wound products that aim to stimulate or replace the ECM. These tissue-engineered products comprise a reconstituted or natural collagen matrix that aims to mimic the structural and functional characteristics of native ECM. When placed in the wound bed, the three-dimensional matrix provides a temporary scaffold or support into which cells can migrate and proliferate in an organized manner, leading to tissue regeneration and ultimately wound closure.

As an example, the Integra® Bilayer Matrix wound dressing (Integra LifeSciences) represents a bilayer of bovine tendon collagen and glycosaminoglycan with a polysiloxane (silicone) membrane. However, this wound dressing has the disadvantage of not adhering to the wound site which requires stapling procedures. Moreover, the biological coating does not fully mimic tissue architecture and lacks important biological active components of the ECM such as MMP cleavage sites, growth factors and cytokines.

Hence, there is still a need for an improved wound care product that mimics the ECM and is easy to apply. The objective of the present invention thus is to provide a wound care product which overcomes at least one of the above mentioned disadvantages.

This problem is solved by provision of a wound care product according to claim 1. Specific embodiments are subject matter of further independent claims.

### Summary of the invention:

In a first aspect the present invention provides a multilayered wound care product comprising:

(a) a layer comprising nanocellulose; and
(b) a hydrogel layer comprising at least one ECM-mimicking structure which is selected from the group consisting of particulate ECM, collagen protein structure, collagen-like protein structure and peptides comprising a matrix metalloproteinase (MMP) cleavage site or a combination thereof.

The wound care product of the present invention has several advantages over wound care products known in the prior art.

By adding a hydrogel layer comprising at least one ECM-mimicking structure it is possible to combine the beneficial properties of the safe and non-noxious nanocellulose layer, which include protection from exterior pollution and bacteria, generation of a humid atmosphere and management of excessive exudate with the reconstructive properties of the ECM.

The nanocellulose layer functions as skin's epidermis, since it protects the wound from infection and controls heat and moisture loss. Furthermore it adheres to the wound site throughout the healing process, so that stapling is not necessary. Once epithelisation is completed and skin has fully regenerated the nanocellulose falls off.

The ECM-mimicking structure of the hydrogel layer closely resembles native ECM and therefor can act as a scaffold for MMPs to bind to and break down collagen in the product. As a result reduced levels of MMPs are released back into wound as collagen matrix breaks down, rebalancing protease and growth factor levels in the wound. Furthermore due to further signalling molecules or parts thereof it induces epithelial cells, fibroblasts and vascular endothelial cells to migrate into the wound layer which then can proliferate.

Also here, the broad spectrum of hydrophilic polymers as basis for the hydrogel allows the selection of the optimal material with regard to costs, biodegradability, strength and flexibility.

Due to the reduction in ECM material, the costs for the wound care product can considerably be reduced while the biological ECM activity is retained.

However, the multilayer of the invention can be provided with additional layers such as, e.g., a covering layer.

By an individual selection of the nanocellulose layer, the hydrogel and the ECM mimicking structure, the wound care product can easily be adapted to the specific requirements of the underlying injury. Hence, it offers an enormous flexibility of application.

Since the wound care product of the invention is based on known components, it can be easily produced in a cost efficient manner.

## Detailed description of the invention

The inventive wound care product may be used as such, as a dressing or in combination with a backing known in the art. For example, the wound care product according to the present invention may be temporarily adhered to a backing. This backing may be removed from the wound care product once applied to the wound or after cellular ingrowth has started.

## Nanocellulose

The layer comprising nanocellulose is preferably a layer that consists of nanocellulose.

The nanocellulose as used in the wound care product of the invention is preferably selected from the group consisting of cellulose nanofibers (CNF), nanocrystalline cellulose and biocellulose produced by bacteria (BNC).

In a preferred embodiment the nanocellulose layer comprises open pores or at least a porosity which enables cells to enter and grow within the scaffold. Porosity is defined as $P=1-p$ (V/M) wherein P is the scaffold porosity, $\rho$ is the density of the polymeric system used, M is the weight and V is the volume of the fabricated scaffolds.

The nanocellulose layer of the invention has a porosity P of more than 50%, more preferably of more than 80%, even more preferably of more than 90% and especially preferably of more than 95%.

## CNF

In one embodiment of the invention the nanocellulose is provided as cellulose nanofibers (CNF).

Cellulose nanofibers (CNF) are defined as nano-sized fibers consisting of a bundle of stretched cellulose chain molecules with long, flexible and entangled cellulose nanofibers of approximately 1-100 nm size with nano-dimension cross sectional structures. They consist of alternating crystalline and amorphous domains. Nanofibers have extremely large specific surface areas, the properties of which are sometimes significantly different from those of the bulk materials. Cellulose nanofibers (CNFs) form long flexible fiber networks with a fibril diameter similar to or larger than CNCs. CNFs can be produced by (2,2,6,6-Tetramethylpiperidin-1-yl)oxyl (TEMPO)-mediated oxidation, multi-pass high-pressure homogenization, enzymatic hydrolysis or direct mechanical fibrillation. The morphologies and dimensions of CNFs can vary substantially, depending on the degrees of fibrillation and any pre-treatment involved. CNFs contain amorphous cellulose and are not as highly crystalline as CNCs.

The defibrillation of CNF needs intensive mechanical treatment. However, according to degree of processing and raw material, chemical pre-treatments are performed before mechanical fibrillation. The processes for isolating CNF consist of disintegration of cellulose fibers along their long axis. They include simple mechanical methods sometimes in combination with enzymatic or chemical pre-treatments. Appropriate pre-treatments of cellulosic fibers promote the accessibility of hydroxyl groups, increase the inner surface, alter crystallinity, and break cellulose hydrogen bonds and therefore, boost the reactivity of the fibers. Mechanical approaches to diminish cellulosic fibers into nanofibers can be divided into refining and homogenizing, microfluidization, grinding, cryocrushing and high intensity ultrasonication. The resulting aqueous suspensions exhibit gel-like characteristics in water with pseudoplastic and thixotropic properties even at low solid content. Three main technologies, namely homogenization (by means of a Manton-Gaulin homogenizer for example), microfluidization and microgrinding, are widely used for the mechanical treatment. The morphological characteristics of the resulting CNFs depend mainly on those of the original fibers. Generally CNFs obtained from primary cell wall fibers are longer and thinner than those obtained from secondary cell wall fibers.

High pressure homogenization (HPH) process includes passing the cellulose slurry at high pressure into a vessel through very small nozzle. High velocity and pressure as well as impact and shear forces on fluid generate shear rates in the stream and decrease the size of fibers to nanoscale. High pressure homogenization can be considered as an efficient method for refining of cellulosic fbers, because of high its efficiency, simplicity and no need for organic solvents.

Microfluidization is another method similar to high pressure homogenization which can be used to produce CNF. Microfluidization includes the use of an intensifier pump to increase the pressure and interaction chamber in order to defibrillate the fibers using shear and impact forces against colliding streams and the channel walls. Smaller CNF with higher surface area can be obtained by increasing the number of passes through the homogenizer. Also, microfluidization can generate nanofibers with a homogenous size distribution.

Another strategy to break up cellulose into nanosize fibers is grinding, for instance with the help of a static and a rotating grind stone, whereas a pulp slurry passes between these two stones during the process. Fibrillation in a grinder proceeds via breaking down hydrogen bonds and cell wall structures by shear forces, thereby individualizing the pulp to nanoscale fibers.

Cellulose nanofibers having an average fiber length of 250 nm or lower and an average fiber diameter of 2 to 5 nm can be produced, for example, by using pulp obtained by hydrolysis treatment and subsequent kraft cooking as a starting material (known as "dissolved pulp by kraft process" or "DKP"), oxidizing the pulp using an oxidant in the presence of a N-oxyl compound or a compound selected from a group consisting of bromide, iodide and mixtures thereof, and then, defibrating the pulp to form nanofibers. The "pulp obtained by hydrolysis treatment and subsequent kraft cooking (DKP)" means pulp obtainable by kraft cooking of a hydrolyzed plant material, such as wood chip, kenaf, hemp, rice, bagasse or bamboo under general conditions.

The type of a plant material used in the preparation of DKP is not particularly limited. Softwood or hardwood chip which is generally used for pulping, kenaf, hemp, rice, bagasse, bamboo or the like may be used. DKP is characterized in that it has been subjected to hydrolysis as a pre-treatment before kraft cooking. The conditions for the hydrolysis treatment are not particularly limited. For example, the treatment may be performed using an autoclave apparatus or the like to contact water or a liquid-phase or vapour-phase mineral acid with a plant material. After the hydrolysis treatment, a neutralization treatment may be performed by using a mixture of sodium hydroxide and sodium sulfide.

In the patent application CA 2888331 A1 suitable methods for producing CNF are disclosed, the disclosure of said document is disclosed herein by reference.

In the preparation of DKP, the conditions for the kraft cooking performed after the hydrolysis treatment are not particularly limited, but the method used in the preparation of common kraft pulp may be used. For example, in a digester, a cooking liquor containing caustic soda (sodium hydroxide) and sodium sulfide as main components may be added to a plant material to first impregnate the plant material with the cooking liquor and then further cook the plant material.

It is generally accepted that oxidation of cellulose with N-oxyl compounds lead to the formation of carboxyl-groups. Oxidation-derived carboxyl groups are negatively charged, so they are mutually repulsive, and their dispersion in water inhibits aggregation of micro fibrils with each other. Consequently, the fiber bundle is released by microfibril units and form cellulose nanofibers, which are single microfibrils of cellulose.

N-oxyl compounds to be used in oxidizing pulp are compounds that may generate nitroxyl radicals. Among these substances, 2,2,6,6-tetramethyl-1-piperidin-N-oxyradical (referred to hereinafter as "TEMPO") and 4-hydroxy-2,2,6,6-tetramethyl-1-piperidin-N-oxyradical (referred to hereinafter as "4-hydroxy TEMPO") are preferred. Derivatives of these substances can also be used.

The amount of an N-oxyl compound may be a catalytic amount sufficient to oxidize pulp so that the obtained oxidized pulp can be formed into nanofibers.

The oxidized pulp is then defibrated to be transformed to cellulose nanofibers. Defibration may be performed by using a mixing/agitating, emulsifying/dispersing device, such as high-speed shearing mixer or a high pressure homogenizer, alone or by a combination of 2 or more types, as necessary. In the process, the size of oxidized pulp (fiber length and fiber diameter) decreases as the fibers loosen and single microfibrils are formed.

To reduce the energy required for defibrating, the oxidized pulp may be subjected to a suitable cutting (also known as "viscosity-reducing treatment") of the cellulose chains (form short fibers from the cellulose chain) before defibration. Such treatment includes, for example, a treatment that radiates ultraviolet rays on oxidized pulp, a treatment that contacts oxidized pulp with hydrogen peroxide and ozone for oxidative decomposition, a treatment that hydrolyzes oxidized pulp with acid, a treatment that hydrolyzes oxidized pulp with alkali, a treatment with enzymes, such as cellulase, or a combination of these treatments.

**Nanocrystalline cellulose**

In one embodiment of the invention the nanocellulose is nanocrystalline cellulose (NCC).

Nanocrystalline cellulose (NCC) is a tightly packed array tightly packed array of microscopic needle-like cellulose crystals. NCC is mainly produced by processing wood pulp. Wood cellulose nanocrystals average 180-200 nm in length with a cross section of 3 - 5 nm and a resulting aspect ratio which varies from 1 to 100, depending on the cellulose source. Nanocrystal dimensions also depend to a certain extent on the hydrolysis conditions used to obtain them.

In a preferred embodiment of the present invention, the nanocrystals of the NCC have an average diameter of less than about 7 nm with substantially all of the nanocrystals have diameters within about 0.5 nm of the average diameter and an aspect ratio of 10 or greater.

The average diameter is preferably less than about 5 nm. In one embodiment, the average diameter is in a range of from about 3 nm to about 7 nm, preferably in a range of from about 3 nm to about 4.9 nm. The aspect ratio is preferably in a range of from about 12 to about 60. Preferably, substantially all of the nanocrystals have diameters within about 0.3 nm of the average diameter.

The NCC as used in the nanocellulose layer preferably has a crystallinity index (CRI) that is 5% or more greater than the CRI of the cellulosic material from which the NCC is made. Advantageously, the CRI may be 7% or more, or even 10% or more than the CRI of the cellulosic material from which the NCC is made. The CRI may be, for example, up to 20% greater, or up to 17% greater than the CRI of the cellulosic material from which the NCC is made. The values of 5% or more, 7% or more or 10% or more may be lower limits of a range in which the upper limit is 20% or 17%. Preferably, the surface carboxylic acid groups are formed by selective oxidation of C6 primary hydroxyl groups of the NCC. Preferably, the degree of oxidation is in a range of from 0.01 to 0.20, more preferably in a range of from 0.02 to 0.2, or in a range from about 0.05 to 0.15, and especially of 0.08.

The most common process for generating nanocrystalline cellulose proceeds via treatment of a cellulose source with a strong mineral acid (such as 64% sulfuric acid), followed by mechanical size reduction. Diverse parent materials can be used, preferably wood pulp. The wood pulp can be prepared from various plants or parts of it such as e.g. cotton, cotton linter, ramie, sisal, tunicate, soft wood and hard wood.

Nanocrystalline cellulose fragments (also known as whiskers, nanowhiskers or nanocrystals) can be generated with variable sizes (widths from 5 to 70 nm and lengths from 100 to several thousand nm). Physical properties of NCC are strongly influenced by source of parent material, the type of acid used in digest (hydrochloric or sulfuric), charge and dimensions.

In an embodiment NCC is produced by controlled acid hydrolysis of cellulose from various sources and preferably from bleached wood pulp. Ground bleached wood pulp is hydrolyzed with 64 % (w/w) sulfuric acid with heating at 45 °C for 25 minutes. The reaction mixture is diluted with water to arrest the hydrolysis and excess acid is removed from the NCC by decantation, centrifugation/washing and dialysis.

In an embodiment, mechanical size reduction processes can be used following the acid digest such as ultrasonic treatment, cryogenic crushing and grinding, and homogenization such as fluidization, which also increase yield. NCC may also be generated from microcrystalline cellulose (MCC) using strong mineral acid hydrolysis followed by separation by differential centrifugation, which results in a narrow size distribution of the NCC.

The optimal conditions can vary considerably, depending on the source of cellulose. In an embodiment the cellulosic material is milled prior to the hydrolysis, usually to pass a to 100 mesh screen. Typically, bleached Kraft pulp from black spruce is milled to pass a 100 mesh screen. An appropriate amount of this pulp, with a water content of about 7% is then added to 60% sulfuric acid kept heated at 60°C. The fluid mixture is stirred for 25 minutes and the hydrolysis is stopped by diluting about tenfold in water. The hydrolysed material is then centrifuged and washed until the pH is $\geq$ 1 and transferred to a dialysis bag. The removal of free acid is thus pursued until the dialysis water remains close to neutral. The material has then passed partly or wholly in suspension, forming a gel. This gel is treated with a sonifier and the resulting liquid is further polished by a mixed-bed ion exchange resin treatment. The final product spontaneously self-assembles to form a chiral nematic liquid-crystalline phase in a certain concentration range, from about 1% to 20% w/w, preferably 2% to 10% w/w. At lower concentrations the suspension is isotropic and at higher concentrations it is a viscous gel which prevents the formation of the chiral nematic structure. The yield is about 60%. The product is now in its acid form so that sulphur content can be measured by titration. The sulphur content is suitably about 0. 4% to 1%, and is typically found to be of the order of 0.7% by dry weight of the solid. The salt forms of the liquid crystal can be obtained by neutralization with NaOH, KOH, etc. The ionic strength of the preparation can be adjusted by addition of electrolyte, but the suspension will precipitate or form a gel above a certain concentration (a salting-out effect), for example, at about 0.05 M NaCl. If the colloidal suspension is allowed to dry on a support surface, for instance in a petri dish at room temperature, it will form a solid in which the order of the liquid crystal has been preserved. The preserved chiral nematic structure is found to be preferentially planar, i.e., with the long axis of the crystallites preferentially parallel to the substrate on which the film was dried.

In a further embodiment well dispersed colloidal suspensions of NCC are obtained by separating the individual NCC particles by a disruptive treatment such as sonication. The stability of the NCC suspensions derives from anionic sulfate ester groups imparted to the cellulose nanocrystal surfaces during hydrolysis. Hydrochloric acid hydrolysis of cellulose in an analogous manner will also produce NCC; however, the NCC particles are electrostatically neutral (without anionic groups attached) and do not form stable aqueous colloidal suspensions. Post-sulfation by sulfuric acid treatment can be used to impart negatively charged groups to this HCl-produced NCC.

The sulfate ester groups are associated with $H^+$ counter ions from the acid hydrolysis, which can be neutralized with a range of inorganic bases (MOH) or organic bases to give salt forms of NCC, (M-NCC) with neutral counter ions other than $H^+$, such as alkali metals and in particular $Na^+$, $K^+$ or $Li^+$, or organic phosphonium ($R_4P^+$) and organic ammonium ions ($R_4N^+$) where each R group which may be the same or different from the other R groups, is an organic chain or group, for example a phenyl group or an alkyl chain of 1 or more, preferably 1 to 4, carbon atoms (e.g., tetraethyl

ammonium ion, $(C_2H_5)_4N^+$). The acidic NCC is designated H-NCC, while the neutral sodium form of NCC is designated Na-NCC. Thermal treatment, both gentle and harsh, has been used to stabilize NCC films, dried by evaporation, against redispersal in water: heating at 35°C for 24 h in a vacuum oven is sufficient for solid H-NCC films evaporated at ambient conditions, although heating overnight at 105 °C and at 80 °C for 15 min have also been used to stabilize spin-coated H-NCC films. NCC films containing $Na^+$ counter ions have been stabilized at 80°C for 16 h; as well as at 105°C for times between 2 and 12 h.

In one embodiment the nanocrystalline cellulose employed in the present invention is derived from cellulose bearing anionic groups, which groups may be associated with cations. In particular, the anionic groups may be sulfate ester groups resulting from hydrolysing of cellulose with sulfuric acid. The cations may, in particular, be alkali metal ions, such as sodium ion or potassium ion.

In a further embodiment, oxidation of biomass from renewable sources to generate NCC with a high degree of carboxylation is conducted in a one-step procedure with ammonium persulfate.

In one embodiment of the invention the NCC is generated by a catalytic reaction process based on breakdown of complex structures into their constituents by reactive oxygen species (ROS) generated from hydrogen peroxide in the presence of a transition metal catalyst, at an acidic pH.

In another embodiment of the present invention, the NCC is produced by a method comprising the following steps: Providing a cellulosic material; contacting the cellulosic material with an inorganic persulfate at an elevated temperature to produce cellulose nanocrystals; and, recovering the cellulose nanocrystals. The inorganic persulfate preferably comprises ammonium persulfate $((NH_4)_2S_2O_8)$, sodium persulfate $(Na_2S_2O_8)$, potassium persulfate $(K_2S_2O_8)$ or a mixture thereof. More preferably, the inorganic persulfate comprises ammonium persulfate, $(NH_4)_2S_2O_8$. In this embodiment, any suitable source of cellulosic materials may be used, for example, vegetative or non-vegetative biomasses. Non-vegetative biomasses include cellulosic materials that have undergone considerable pre-treatments, for example, cellulose from papers and microcrystalline cellulose (MCC). In the described embodiment NCC may be produced in one step from vegetative biomass. Thus, the cellulosic material preferably comprises vegetative biomass, more preferably raw vegetative biomass. Any suitable vegetative biomass may be used, for example, one or more of hemp material (e.g. raw hemp, pectate-lyase treated hemp), flax material (e.g. raw flax, pectate-lyase treated flax), triticale material, wood sources (e.g. wood pulp, cardboard) and agricultural residues. More preferably, the cellulosic material comprises one or more of hemp or flax material. The process is conducted at an elevated temperature. Preferably, the elevated temperature is in a range of from about 45°C to about 80°C, for example about 60°C. The persulfate is preferably provided in an aqueous solution. The aqueous solution preferably has a concentration of persulfate in a range of from about 0.5 M to about 2.0 M, more preferably in a range of from about 0.5 M to about 1.0 M, for example about 1.0 M. Preferably, the persulfate is stirred with the cellulosic material. Preferably, contacting the cellulosic material with persulfate is performed for a period of time in a range of from about 5 hours to about 24 hours, for example about 16 hours.

In a preferred embodiment of the invention the NCC is provided as a film. In a preferred embodiment this film is produced from an aqueous suspension of NCC whereby the process is more preferably performed as follows: The aqueous NCC suspension is typically spaced from a source of heat whereby a heat transfer zone is disposed between the suspension and the source of heat. This heat transfer zone may typically be air. Suitably, the suspension itself is in the form of a thin liquid layer. Applying heat in this way from the heat source through the heat transfer zone results in a relatively uniform transfer of heat from the source to the suspension. With the evaporation of water from the suspension, there is thereby formed a film.

The heat transfer rate of the heating zone is suitably one sufficient to permit evaporation of water from the suspension with formation of the required solid film. In particular, the evaporation time is suitably and sufficient to permit the self-assembly of the NCC particles into a chiral nematic organization; a typical suitable range of time for the evaporation is 4 to 6 hours.

The solid film produced may be a solid film of the NCC, or may be a plasticized film of NCC achieved by including a plasticizer such as polyvinyl alcohol in the suspension.

In another embodiment of the invention, material such as plasticizers, polymer resins, or reinforcing agents (woven or non-woven fibres of glass, carbon, wood, etc.) can be added to the dispersion. For instance, a plasticizer such as glycerol will make the films more pliable; a water soluble resin, such as a melamine resin will have the same effect, as well as a strengthening effect. Such a resin may or may not be cross-linked to the crystallites.

**BNC**

In one embodiment of the invention the nanocellulose is a biocellulose produced by a microorganism, which preferably is a bacterium of the genus *Acetobacter.*

In one embodiment of the invention two or more strains of *Acetobacter* might be used in combination to manufacture matrices of varying nanocellulose strands, which may be brought together to form nanocellulose of varying layers, levels and controlled three dimensional structures.

In one embodiment, the present invention comprises at least one multi-ribbon biocellulose produced by, e.g., *Acetobacterxylinum.*

A further embodiment of the present invention comprises at least one multi-ribbon biocellulose which is produced by *Acetobacter xylinum* strain NQ-5 under conditions in which the biocellulose ribbons are deposited at the liquid gas interface and/or under shaking conditions.

Certain strains are known in prior art that yield cellulose with reversal of direction of cellulose ribbon extrusion. The reversal of direction of cellulose ribbon extrusion results from the cellulose-producing microorganism shuttling, at least periodically, first in one direction and then in the other direction along a length of an earlier-deposited cellulose ribbon to add another cellulose ribbon thereto and produce a cellulose ribbon-bundle having a width of at least two cellulose ribbons. The cellulose producing microorganism of the present invention may be at least one of the genuses Sphaerotilus, Pseudomonas spp., Alcaligenes spp., Salmonella spp., Agrobacterium spp., Rhizobium spp., any cyanobacterium that produces cellulose or is genetically modified to produce cellulose such as Nostoc, Scytonema, Synechococcus, Agmenellum or Anabaena. Preferably, the cellulose producing microorganism of the present invention may be at least one of the genus Acetobacter and more preferably of the species *Acetobacter xylinum* or *Acetobacter pasteurianus.* Among preferred *Acetobacter xylinum* strains are strain NQ5, H1A; H1B; H1C; H2A; H2B; H5C; H5D; H6A; H6C; H7B; H8C; H8G; H9D; H10C; H14B; H15A; and H15B. *Acetobacter xylinum* strain NQ5, has particularly useful characteristics and has deposit No. ATCC 53582 with the American Type Culture Collection, Rockville, MD.

One example of microorganisms that produce a nanocellulose include members of the genus Acetobacter or bacteria, microorganisms or organisms or tissues that have been transformed (permanently or transiently) with one or more genes capable or required for manufacturing cellulose and strains or sub-strains related to or derived therefrom. The nanocellulose may be a cellulose derivative, such as carboxymethyl-nanocellulose, methyl-nanocellulose, hydroxyethyl-nanocellulose, hydroxypropyl-nanocellulose and hydroxypropylmethyl-nanocellulose or mixtures or combinations thereof. The fibrous organic substrate may be a microfibrillar cellulose that is wet, partially wet, dry, anhydrous, hydrated, coated or uncoated at a submicron thickness.

Yet another aspect of the invention provides a method for increasing cellulose production in a recombinant microorganism, which method comprises transforming a suitable microorganism with a vector comprising at least one gene derived from a bacterial cellulose synthase operon, and culturing the transformed microorganism under conditions suitable for production of cellulose. In an embodiment, the chromosomal cellulose synthase promoter can be replaced with a heterologous promoter to overexpress an intrinsic cellulose synthase operon at the chromosomal level.

A wide variety of cellulose operons and promoter systems may be used in order to facilitate a nanocellulose production in a genetically modified organism, e.g., the cellulose operon acsABCD from NQ5 under the control of an PrbcL promoter from *Synechococcus leopoliensis,* a portion of the cellulose operon sufficient to express bacterial cellulose that includes the acsAB genes from the cellulose synthase operon of *Acetobacter sp.*

In a preferred embodiment, the portion of the cellulose operon sufficient to express biocellulose may include the acsAB genes from the cellulose synthase operon of the gram negative bacterium *Acetobacter xylinum* or from the Acetobacter multiribbon strain NQ 5. It has been found that it is possible to manufacture cellulose with a lower crystallinity than wild-type bacterial cellulose, amorphous cellulose. In one embodiment of the present invention, the cellulose genes are from mosses (including Physcomitrella), algae, ferns, vascular plants, tunicates, and combinations thereof. In yet another non-exclusive embodiment, the cellulose genes are selected from gymnosperms, angiosperms, cotton, switchgrass and combinations thereof. The skilled artisan will recognize that it is possible to combine portions of the operons of bacterial, algal, with fungal and plant cellulose genes to maximize production and/or change the characteristics of the cellulose.

In an embodiment the bacterial nanocellulose is produced by at least one genetically modified organism being preferably a cyanobacterium, which is transformed with a vector for expression of a portion of the cellulose operon sufficient to express bacterial cellulose operon that includes a microbial cellulose operon, e.g., the acsAB gene operon, under the control of a promoter that expresses the genes in the operon in cyanobacteria. The skilled artisan will recognize that the vector may combine portions of the operons of bacterial, algal, fungal and plant cellulose operons to maximize production and/or change the characteristics of the cellulose and may be transfer and/or expression vector.

As used herein, the term *Acetobacter* refers to a genus of microorganisms, and in particular, to the members of that genus that produce cellulose. Although a number of microorganisms fitting this description are known, their taxonomic classification has been subject to debate. Thus, any cellulose producing strain of *Acetobacter* whether classified as *Acetobacter aceti* subsp. *xylinum, Acetobacter pasteurianus* or otherwise, that has the characteristics of stability under agitated culture conditions as further explained below, is considered to be within the scope of the invention.

The cellulose ribbon-bundles produced by the cellulose-producing microorganism of the present invention may be characterized as comprising antiparallel cellulose ribbons having beta-1, 4 linkages.

In an embodiment, microorganisms producing nanocellulose do so by proceeding in a first direction alternating with cellulose ribbons having beta-1,4 linkages proceeding in an opposite direction. This alternating structure results from reversals in the direction of cellulose ribbon extrusion from a microorganism traveling along a previously deposited cellulose ribbon and depositing a new cellulose ribbon which becomes hydrogen-bonded to the adjacent earlier deposited cellulose ribbon. The cellulose of the present invention is preferably produced by a cellulose-producing microorganism aerobically cultivated in an aqueous nutrient medium having a pH between about 3 and about 7 and at a temperature between about 20 °C and about 40 °C.

In an embodiment the cellulose-producing microorganism are capable of repeated reversals of direction of cellulose ribbon extrusion. Such directional reversals of extrusion result in said cellulose-producing microorganism shuttling, at least periodically, first in one direction and then in the other direction along a length of one or more earlier extruded cellulose ribbons to add another cellulose ribbon thereto. This process produces a cellulose ribbon-bundle being stronger than that produced by non-shuttling microorganisms. These ribbon-bundles have a width of at least two, and preferably three or more, cellulose ribbons. The cellulose thus produced may be collected for use or further processing, if desired. The microorganisms and conditions described above may be used to form this product cellulose comprising cellulose ribbon-bundles with cellulose ribbons of alternating antiparallel orientation, also described above. These antiparallel ribbon alignments result in glucan chain interrelationships reminiscent of cellulose II structure. This cellulose product of a reversal-type cellulose producing microorganism may thus be a polymorph of cellulose I and cellulose II. The antiparallel ribbon alignment should result in one additional inter-ribbon hydrogen bond for each pair of interacting glycosidic units. This additional hydrogen bond, along with the greater numbers of ribbons in a bundle, is consistent with a apparent greater physical strength observed.

In an embodiment the manufacturing of the bacterial nanocellulose includes the step of growing at least one layer of a bacterial nanocellulose substrate in a vessel that serves as a template for the shape of the dressing. In an embodiment, this template may be preformed based on, e.g., a scanned image or other measurements of the wound location. One or more wound dressings may be prefabricated to serve as replacements for the respective prior dressing during the recovery of the wound site, e.g. in case of grafting operations in which the wound may take, days, weeks or even months to heal and/or for patients that have slow wound healing processes.

In an embodiment of the invention, the wound dressing may be grown, assembled or synthesized on a substrate that provides a template for the shape of the dressing. In a preferred embodiment, the dressing comprises reservoirs for the external addition of at least one active agent. In a preferred embodiment, the substrate that provides a template for the shape of the dressing also provides elements for the formation of at least one reservoir for the growth of cells or the deposition of at least on biologically active agent in the nanocellulose.

Using the present invention, the nanocellulose can also be designed to provide diversity in structure and functionality because before, during or even after synthesis it is possible to laminate or sandwiched the strings, sutures, sheets and/or membranes either from the same organism, or from different organisms. The manufacturing process can be applied after synthesis; however, the actual use of two or more nanocellulose manufacturing strains may be used at the same time to generate hybrid membranes of cellulose with physical features that are different from either grown alone. Methods of cellulose molding during synthesis are taught, generally as published in EP Application No. EP0186495, which teaches a process for microbial cellulose production; relevant techniques, materials and methods incorporated herein by reference. A liquid culture of cellulose-producing microorganism and a structure are taught. The structures may be made in the presence of an oxygen-containing gas to create, if required, a gas permeable material. Otherwise, the structure is generally not gas permeable. Agents may be added during synthesis to alter the configuration of microbial cellulose. These same techniques may be applied to the nanocellulose of the present invention.

In one embodiment the bacterial nanocellulose of the present invention is synthesized by bacteria, preferably the bacteria *Acetobacter xylinum,* and was recovered from inoculation flasks and propagated via continued inoculation and incubation for linear growth in subsequent flasks and carboys of optimized media to attain the desired volume of microbially derived cellulose. The media is comprised of nutrients such as sucrose, ammonium sulfate, sodium phosphate, magnesium sulfate, citric acid, acetic acid and trace elements resulting in a growth media having a pH of about 4.0 to about 4.4. The sterilized media is inoculated from propagation cultures of *A. xylinum* and filled into bioreactor trays at the appropriate volume to yield a final cellulose to water ratio of about 90% to 95% water to about 5% to 10% cellulose. The bioreactor trays are sealed and incubated in a controlled environment at 30°C $\pm$ 2° until growth of a pellicle of bacterial nanocellulose is complete. The pellicles are removed from the bioreactor trays and are chemically treated to remove bacterial by-products and residual media. A caustic solution, preferably sodium hydroxide at a preferable concentration of about 0.1 M to 4 M, is used to remove viable organisms and pyrogens (endotoxins) produced by bacteria from the pellicle. The treated pellicles are then rinsed with filtered water to reduce microbial contamination (bioburden).

Further processing of the present invention continues with the use of a water-miscible organic solvent selected from the group consisting of methanol, ethanol, propanol, isopropanol, acetone and mixtures thereof to dehydrate the cellulose. Without being bound to any one theory, it is believed that soaking the compressed films in a water-miscible organic solvent cross-links the cellulose fibers, thereby yielding a product having increased tensile strength, reduced elongation (stretch) and increased suture retention when used as an implantable medical device for various surgical procedures.

In an embodiment the present invention undergoes depyrogenation by using a heated caustic solution (0.1 M to 4 M sodium hydroxide) known to destroy endotoxins that may be present due to bacteria or cross-contamination from materials exposed to pyrogens. The material is then gamma irradiated at doses sufficient to destroy microorganism contamination by pre-determined sterility assurance levels based on bioburden levels (the amount of microorganisms typically present on the non-sterile material.) Samples were gamma irradiated at a dose of about 35kGy. It can be concluded that the material can be depyrogenated with a strong alkaline sodium hydroxide solution at an elevated temperature and that it can withstand gamma sterilization without any significant affect to mechanical properties.

The cellulose pellicle is subjected to a series of chemical wash steps to convert the raw cellulose film into a medical grade and non-pyrogenic wound dressing material. Typical processing uses hydroxide solutions at concentrations of 1-20% by weight. Preferably, sodium hydroxide is used at a concentration of not less than 3% and most preferably about 3% to about 5% in order to acetylate and eventually dissolve the cells. In addition, the present invention provides hydrogen peroxide washing capable of bleaching and sterilizing the pyrogen free films. Concentrations of about 0.05% to about 10% peroxide by weight are useful to effect whitening of the films. Preferably the amount of peroxide used in about 0.1 % to about 0.5%.

The cellulose fibrils produced by these microorganisms, although chemical resembling, in many aspects, cellulose produced from wood pulp, are different in a number of respects. Chiefly among the differences is the cross sectional width of these fibrils. The cellulose fibrils produced by *Acetobacter* are usually two orders of magnitude narrower than the cellulose fibers typically produced by pulping birch or pin wood. The small cross sectional size of these *Acetobacter* -produced fibrils, together with the concomitantly greater surface area than conventional wood-pulp cellulose and the inherent hydrophilicity of cellulose, leads to a cellulose product having unusually great capacity for absorbing aqueous solutions.

A use of *Acetobacter xylinum* to coat synthetic fibers with microbial cellulose is disclosed in U.S. 4,378,431 A1, which is incorporated by reference herein.

In a preferred embodiment, the invention provides a bacterial nanocellulose that is bioresorbable. Variable degrees of oxidation affect the rate of resorption. For example, the microbial cellulose may be subjected to oxidation at various degrees to render it bioresorbable. This resorption can be tailored to individual needs so that voids are not created by the material degrading too quickly. Preferably, the degree of oxidation is between 10 and 30 %.

The degree of oxidation of the bacterial nanocellulose is achieved by varying a factor such as concentration and volume of oxidizing agent, a ratio of oxidizing agent to microbial cellulose, reaction temperature and duration, and combination thereof. For example, the molarity of oxidizing agent may be in the range of 0.005M to 0.5M, and the temperature may be from about 5 to 50°C. Preferably, the microbial cellulose may be oxidized for at least 30 minutes, or 1, 6, 12, or 24 hours, or longer. In a preferred embodiment, the microbial cellulose may be oxidized with a solution containing sodium periodate such as sodium meta periodate and optionally, a supporting electrolyte such as NaCl. In a specific embodiment, the microbial cellulose paste may be oxidized by incubating the paste in 80 mM $NAIO_4$ for 18 to 20 hrs at 30°C.

The final level of oxidation for the microbial cellulose can be tailored to the specific product application, but preferably, the level of oxidation is sufficient to render the microbial cellulose bioresorbable at a desired rate of degradation including rates ranging from as little as one day to over one year. In other words, the oxidized microbial cellulose can be prepared so as to be resorbable in about one, two, three, four, or five days, about one, two, or three weeks, about one, two, three, four, five, six, seven, eight, nine, ten, or eleven months, or about one or two years.

## Hydrogel

According to the invention the hydrogel layer is a water-containing hydrogel constituted by one or more isolated hydrophilic polymers. The term "isolated hydrophilic polymer" refers to the fact that the one or more polymer is a polymer isolated as based on a chemical synthesis (i.e. a synthetic polymer) or a polymer isolated from an in vitro or in vivo source (i.e. a natural polymer). Typically, the de-novo synthesized polymer represents a synthetic polymer with monomers such as ethylene glycol or acrylic acid. In contrast a polymer isolated from an in vitro or in vivo source represents a natural polymer and includes polysaccharides, peptides or proteins. Due to the aforementioned definition of the term "isolated hydrophilic polymer" the term hydrogel layer excludes an extracellular matrix as a hydrogel since its proteins (such as collagen) which are represent hydrophilic proteins, are not isolated before constitution.

In general, hydrogels comprise three-dimensional (3D) crosslinked networks of hydrophilic polymers that swell in water. Water can penetrate in between the polymer chains of the polymer network, subsequently causing swelling and the formation of a hydrogel. Hydrogels are superabsorbent (e.g. they can contain over 99% water) and possess a degree of flexibility which is, due to their significant water content, very similar to natural tissue.

In certain embodiments an important attribute of the hydrogels used herein is their large water content, typically greater than about 95% resulting in high permeability for oxygen, nutrients, and other water-soluble metabolites.

In certain embodiments the hydrogel comprises at most about 90% water w/v, or at most about 95% water w/v, or at most about 96% water w/v, or at most about 98% water w/v, or at most about 99% water w/v.

The percentage of water and the degree of swelling of the materials can, depending on the degree of crosslinking of the hydrogel, be varied over a wide range by varying the individual parameters.

The scaffold of the hydrogels according to embodiments of the invention, with widely varying physical properties, for example stiffness and hydration, forms the structural, supporting and protective function of the material

In one preferred embodiment, the stiffness of the hydrogel is between 50 Pa to 1 kPa which is similar to adipose and neural tissue.

A substantial advantage is the modular character of the hydrogels. The gel is formed by chemically defined components and can therefore be adjusted to wide range of conditions with a high degree of precision. This flexibility enables the design and construction of sophisticated bioactive and biocompatible devices. Biofunctionalization, for example control of cell adhesion by means of RGD peptides, direct crosslinking or crosslinking with enzyme-cleavable peptides and loading with growth factors can be varied independently of one another and over a wide range.

The physical properties can moreover be varied as required via the quality and quantity of the coupling points within the network of the hydrogels. The quality of coupling is determined conceptually by means of the coupling mechanisms used and/or via the modified components, whereas the quantity of coupling is determined by the relative proportions of the components and the activation parameters.

These properties mean that the bioactive hydrogels can be used long-term, for periods from several weeks to months.

One interesting way to design the aforementioned hydrogel systems is to conjugate organic macromolecules and synthetic polymers. The organic macromolecules, for example peptides, oligosaccharides or oligonucleotides, can be either of synthetic or biological origin. Synthetic peptides and DNA can for example originate from solid phase synthesis. It is very important that bio-macromolecules utilized for the production of hydrogels intended to be used for medical purposes have non-toxic properties and low immunogenicity. Both covalent and non-covalent methods can be used for cross-linking organic macromolecules and synthetic polymers, wherein the non-covalent methods are of particular interest due to the possibility to produce a great variety of different gels. Moreover, non-covalent, self-organizing systems made from organic macromolecules and synthetic polymers enable the embedding of cells in a matrix system without the need for chemical reactions.

**Hydrogel - synthetic polymers**

In one embodiment of the invention, the hydrogel comprises at least one synthetic polymer.

In one embodiment of the invention the hydrogel layer comprises a synthetic polymer selected from the group consisting of polyglycolic acid (PGA), polylactic acid (PLA); polyethylene glycol (PEG) polyvinyl alcohol, poly(N-isopropyl acrylamide), poly(lactic-co-glycolic acid) (PLGA), polyethylene glycol diacrylate (PEGDA), a polyethylene glycol divinyl sulfone, a polyethylene glycol bismaleimide, a polyethylene glycol dimethyacrylate, a polyethylene glycol (methyl)acrylamide and a polyethylene glycol di(halo)acetate and copolymers or mixtures thereof.

In a preferred embodiment of the invention, the hydrogel comprises at least one linear, star, comb or branched PEG or a combination thereof. The well-known resistance of PEG materials to nonspecific protein adsorption reduces unwanted, uncontrolled interactions with biomolecules, whereas interaction with therapeutically active cells and microorganisms can be controlled and regulated by targeted modification of the at least one PEG with peptides or proteins specifically binding to biological target structures. One example for such PEG-modifying peptides are cell adhesion proteins (RGD peptides). Since PEG hydrogels are nontoxic, non-immunogenic and inert to most biological molecules (e.g., proteins), they are also approved by the regulatory authority for various clinical uses.

In an especially preferred embodiment of the invention, the hydrogel comprises star-branched polyethylene glycol (star-PEG) with functionalized end groups.

In one embodiment of the invention, the hydrogel comprises a star-PEG with a molecular weight of from 5,000 Da to 40,000 Da, and particularly from 8,000 Da to 20,000 Da, such as 10,000 Da.

In a further embodiment of the invention, the hydrogel comprises a polyethylene glycol (PEG) having a photopolymerizable functional group. The photopolymerizable functional group may comprise at least one selected from the group consisting of acrylate, methacrylate, coumarin, thymine, and cinnamate. In one embodiment of the invention, a photopolymerization initiator serves to generate a radical for initiating photopolymerization of the PEG and preferably generates a radical in response to visible light of a wavelength of 350 nm to 750 nm.

Poly(hydroxyethyl methacrylate) (PHEMA) represents another preferred synthetic polymer which is characterized by desirable mechanical properties, optical transparency, and stability in water. Like PEG, various modifications can be made to generate PHEMA derivatives to modify its properties. For example, dextran-modified PHEMA gels have been synthesized to modulate the degradation properties of a hydrogel. Copolymerization of PHEMA monomers with other monomers, such as methyl methacrylate, can be used to modify properties such as swelling and mechanical properties.

In another embodiment PVA is used as structure-forming constituent of the hydrogel. PVA hydrogels are stable, elastic gels that can be formed by physical crosslinking methods (e.g., repeated freezing and thawing process), chemical crosslinking methods (e.g., glutaraldehyde, acetaldehyde, formaldehyde, and/or other monoaldehydes), irradiative crosslinking mechanisms (e.g., electron beam and/or gamma irradiation), and/or photo-crosslinking mechanisms. Physically crosslinked versions of PVA hydrogels are biodegradable, and thus can be used for various biomedical applications.

## Hydrogel-oligo/polysaccharides

In another embodiment of the invention the hydrogel of the hydrogel layer further comprises a highly negative oligo- or polysaccharide, whereby the hydrogel is preferably construed as an oligosaccharide/peptide/polymer system.

In a further embodiment said highly negative oligo- or polysaccharide is selected from the group consisting of heparin, dextran sulfate, α-cyclodextrin sulfate, β-cyclodextrin sulfate, γ-cyclodextrin sulfate, α-cyclodextrin phosphate, β-cyclodextrin phosphate, and γ-cyclodextrin phosphate.

In an embodiment of the invention, the hydrogel comprises cyclodextrin phosphate, preferably α-cyclodextrin phosphate, ß-or y-cyclodextrin phosphate of pharmaceutical grade, whereas the degree of phosphorylation can vary between three phosphate groups per molecule and fully phosphorylated cyclodextrin molecules.

In an embodiment of the invention the hydrogel matrix comprises heparin derived from mucosal tissue of porcine intestine or bovine lung as an oligosaccharide.

In another embodiment of the invention, the hydrogel comprises heparin of pharmaceutical grade.

In a preferred embodiment the highly negative oligosaccharide is dextran sulfate with a molecular weight between 4 to 600 kDa.

In a preferred embodiment of the invention, the at least one highly negatively charged oligo- or polysaccharide is conjugated to at least one synthetic polymer.

In certain embodiments of the invention, the hydrogel comprises a hyaluronan (or a functionalized/derivatized hyaluronan) and a gelatin (or a functionalized/derivatized gelatin).

In certain embodiments the hyaluronan and the gelatin are each thiolated. In certain embodiments the hyaluronan and the gelatin have each been thiol-modified using carbodiimide mediated hydrazide chemistry. In certain embodiments a hyaluronan sodium salt is used. In certain embodiments an acrylated hyaluronic acid is used. In certain embodiments the hyaluronan derivative and gelatin derivative are crosslinked. In some embodiments, the gel-forming material is based on chemically-modified hyaluronic acid.

Another example of a crosslinked hydrogel comprises thiol-modified hyaluronan and thiol-modified heparin (which can ionically bind a wide variety of growth factors and slowly releases them over time) reacted with a thiol-reactive crosslinker such as polyethylene glycol diacrylate. Thiol-modified heparin allows the slow release of growth factors (GFs) within an easily customizable environment.

In certain embodiments, the hydrogel is just formulated with a thiolated hyaluronan and a thiolated heparin. This hydrogel is a synthetic extracellular matrix (ECM) that can be crosslinked in situ. Unlike an animal-derived extracellular matrix (ECM), the hydrogel is chemically defined and is typically non immunogenic. In certain embodiments the hydrogel is formulated with a thiolated hyaluronan, a thiolated heparin, and a thiolated gelatin. In various embodiments the immobilized heparin in the hydrogel mimics the heparin sulfate proteoglycans normally present in the extracellular matrix (ECM). It also helps protect growth factors from proteolysis and slows their release to attached cells. This reduces the amount of growth factor required to achieve stimulation of cell growth or differentiation when compared to the use of free growth factor in media.

Another example of a crosslinked hydrogel comprises thiol-modified hyaluronan and thiol-modified heparin reacted with a thiol-reactive crosslinker such as polyethylene glycol diacrylate. Examples also include hyaluronan sodium salt solution mixed with ADH and carbodiimide reagent (EDC); or acrylated hyaluronic acid dissolved in a TEA-buffered solution and mixed cross-linker peptides such as GCRDGPQGIWGQDRCG (SEQ ID No. 1), or GCRDGDQGIAGFDRCG (SEQ ID No. 2), and the like.

In another embodiment of the invention, the hydrogel comprises at least one natural polymer from the group consisting of carboxyl-methyl cellulose, herparan sulfate and chitosan.

In a preferred form the hydrogel is build up from chitosan. Chitosan is a linear polysaccharide composed of randomly distributed β-(1-4)-linked D-glucosamine (deacetylated unit) and N-acetyl-D-glucosamine (acetylated unit). It is made by treating shrimp and other crustacean shells with the alkali sodium hydroxide. Dissolved chitosan can be crosslinked by increasing pH, by dissolving in a nonsolvent, or by photo-crosslinking. Chitosan can be degraded by the lysosome and is therefore biodegradable. Chitosan gels have been used for many applications including drug delivery. In specific embodiments, a polymer to be included in a precursor solution in accordance with the present invention is N,O-carboxylmethyl chitosan.

Other illustrative and non-limiting hydrogels include, for example, calcium crosslinked alginate, photocrosslinked alginate, and collagen hydrogels (see, e.g., Krebs et al. (2009) J Am. Chem. Soc., 131 (26): 9204-9206). Gils, et al. (2010 Am. J Biomed. Sei. 2(4), 373-383 describe modification of xanthum gum (XG) polysaccharide to produce drug delivery hydrogels. In particular, they prepared XG-g-poly [HEMA-co-AA] superporous hydrogel (SPH) through chemical cross-linking by graft copolymerization of 2-hydroxyethylmethacrylate (HEMA) and acrylic acid (AA) on to XG via redox initiator system of ammonium persulfate (APS) and N, N, N',N'-tetramethylethylenediamine (TMED), in the presence of N,N'-methylene bisacrylamide (MBA) crosslinking agent, sodiU111 bicarbonate foaming agent, a triblock copolymer of polyoxyethylene/polyoxypropylene /polyoxyethylene as a foam stabilizer. Oxidized alginate and gelatin (e.g., periodate oxidized sodium alginate) hydrogels are described by Balakrishnan and Jayakrishnan (2005) Biomaterials 26(18): 3941-3951.

In certain embodiments the heparin derivative is linked to the hyaluronan derivative and/or to the gelatin derivative with a reagent selected from the group consisting of a polyethylene glycol diacrylate (PEGDA), a polyethylene glycol divinyl sulfone, a polyethylene glycol bismaleimide, a polyethylene glycol dimethyacrylate, a polyethylene glycol (methyl)acrylamide, and a polyethylene glycol di(halo)acetate. In certain embodiments the heparin derivative is linked to a hyaluronan derivative and/or to the gelatin derivative with a polyethylene glycol diacrylate (PEGDA) linker.

In certain embodiments the derivatives of hyaluronan and gelatin are crosslinked with a divalent or polyvalent electrophile. In certain embodiments the derivative of hyaluronan and gelatin are crosslinked with a reagent selected from the group consisting of a polyethylene glycol diacrylate (PEGDA), a polyethylene glycol divinyl sulfone, a polyethylene glycol bismaleimide, a polyethylene glycol dimethyacrylate, a polyethylene glycol (methyl)acrylamide, and a polyethylene glycol di(halo)acetate. In certain embodiments the hyaluronan derivative h is linked to the gelatin derivative with a divalent or polyvalent electrophile. In certain embodiments the hyaluronan derivative is linked to the gelatin derivative with a reagent selected from the group consisting of a polyethylene glycol diacrylate (PEGDA), a polyethylene glycol divinyl sulfone, a polyethylene glycol bismaleimide, a polyethylene glycol dimethyacrylate, a polyethylene glycol (methyl) acrylamide, and a polyethylene glycol di(halo)acetate. In certain embodiments the hyaluronan derivative and gelatin derivative are crosslinked with a polyethylene glycol diacrylate (PEGDA) linker. In certain embodiments the hydrogel further comprises a heparin derivative. In certain embodiments the heparin derivative is a thiol-modified heparin. In certain embodiments the heparin derivative is linked to the hyaluronan derivative and/or to the gelatin derivative. In certain embodiments the heparin derivative is linked to the hyaluronan derivative and/or to the gelatin derivative with a divalent or polyvalent electrophile. In certain embodiments the heparin derivative is linked to the hyaluronan derivative and/or to the gelatin derivative with a reagent selected from the group consisting of a polyethylene glycol diacrylate (PEGDA), a polyethylene glycol divinyl sulfone, a polyethylene glycol bismaleimide, a polyethylene glycol dimethyacrylate, a polyethylene glycol (methyl)acrylamide, and a polyethylene glycol di(halo)acetate. In certain embodiments the heparin derivative is linked to the hyaluronan and/ or to the gelatin with a polyethylene glycol diacrylate (PEGDA) linker.

**Hydrogel - crosslinking agent**

In one embodiment of the invention, the hydrogel comprises at least one synthetic polymer as a cross-linking reagent. In certain embodiments the hydrogel comprises a divalent or polyvalent (e.g., trivalent, tetravalent, pentavalent, etc.) electrophile as a crosslinking reagent. Further crosslinking agents are preferably selected from the group consisting of polyfunctional alcohols, alpha hydroxyketones, bis-acyl-phosphine-oxides, vinylpyrrolidones, dithiols, diamines, ammonia persulfates, phenylacetophenones, benzoyloxysuccinimides, carbodiimides, maleimidopropionic acids, propionyl hydrazides, phenyl isocyanates, succinimidyl butyrates, aminobenzoates, succinimidyl propionates, succinimidyl iodoacetates, succinimidyl hexanoates, succinimidyl tartrates, succinimidyl propionates, succinimidyl suberates, succinimidyl glutarates, maleimidoethanes or maleimidobutanes.

A preferred hydrogel layer for use in the present invention further contains bioactive molecules which might be already present in the hydrogel or alternatively added to the hydrogel after preparation. For example, the hydrogel may contain at least one compound from a group including fibroblast growth factor-2 (basic FGF), transforming growth factor-beta (TGF-beta) platelet derived growth factor (PDGF), cartilage derived growth factor (CDGF), epidermal growth factor (EGF), transforming growth factor alpha (TGF-alpha), thrombospondin, osteopontin, angiotensin converting enzyme (ACE), bone morphogenetic protein (BMP), NGF (nerve growth factor), tumor necrosis factor alpha (TNF-$\alpha$), acidic fibroblast growth factor (aFGF), hepatocyte growth factor (HGF), insulin-like growth factors 1 and 2 (IGF-1 and IGF-2), stromal derived factor 1 alpha (SDF-1 alpha), ciliary neurotrophic factor (CNTF), neurotrophin-3, neurotrophin-4, neurotrophin-5, pleiotrophin protein (neurite growth-promoting factor 1), midkine protein (neurite growth-promoting factor 2), brain-derived neurotrophic factor (BDNF), tumor angiogenesis factor (TAF), corticotrophin releasing factor (CRF), interleukin-8 (IL-8), granulocyte-macrophage colony stimulating factor (GM-CSF), and/or vascular endothelial growth factor (VEGF). Further preferred is that the ECM material used in the present invention contains additional bioactive components including, for example, one or more of collagens, glycosaminoglycans, glycoproteins and proteoglycans. The ECM material may further include the basement membrane or parts thereof, which are typically made up mostly of type IV collagen, laminins and proteoglycans.

In a preferred embodiment of the invention the hydrogel layer has a modulus of elasticity of at least 10 Pa.

In another embodiment of the invention the hydrogel is a self-organizing hydrogel matrix.

In a specific embodiment of the invention, the desired properties of the hydrogels are realized by the synthesis of a hybrid material from a biologically active component, namely the natural ECM component heparin and furthermore a synthetic, star-branched polyethylene glycol with functionalized end groups (star-PEG). This hybrid material is also denominated as "heparin/star-PEG hydrogel" herein. The adequate presentation of heparin in the network allows reversible binding and release of therapeutically relevant signal molecules.

For this embodiment of the invention, heparin with a molecular mass weight of 14,000 Da is preferred.

For the heparin/star-PEG hydrogel it is preferred that heparin and star-PEG are bound covalently by amide bonds formed by a reaction between carboxyl groups of heparin activated directly by 1-ethyl-3-(3-dimethylaminopropyl) carbodiimides/N-hydroxysulfosuccinimide (EDC/s-NHS) and terminal amino groups of the star-PEG.

The ratio of heparin to star-PEG determines the degree of crosslinking and therefore the physical properties of the resultant gel materials. In certain embodiments of the invention, molar ratios of star-PEG to heparin from 1 : 1 to 6: 1 are used for this.

In another embodiment, the heparin is bound covalently to the polyethylene glycol via short enzyme-cleavable peptide sequences as the crosslinking molecule. For this purpose it is preferred to use MMP-cleavage sites. As a result the hydrogel is degraded over the time by the matrix metalloproteases (MMP) entering the hydrogel layer from the wound.

On the one hand, those polymers have been developed as chemical structures with minimal interaction with biological systems. For example, they are often not recognized by the immune system as antigens, thereby complications of immunogenicity can be avoided. On the other hand this advantage of low immunogenicity and the accompanied lack of biological function is a disadvantage in respect to the desired ability to communicate with living systems, for example in terms of dynamics and signalling-processes in the extracellular matrix (ECM).

The choice of the polymer and the oligosaccharide may result in different gel properties, including variations in the flow behaviour, the gelation-state, the gelation rate as well as in an adjustable affinity of oligosaccharide-interacting bioactive proteins, including growth factors or morphogen lead.

**ECM-mimicking structure**

According to the invention the ECM-mimicking structure is one or more of the following: particulate ECM, collagen protein structure, collagen-like protein structure and peptide comprising a matrix metalloproteinase (MMP) cleavage site.

The particulate ECM as directly deriving from natural ECM comprises the relevant components for providing an ECM-like biological function. The further components, namely the collagen protein structure, the collagen-like protein structure or the peptide comprising a matrix metalloproteinase (MMP) cleavage are modified to exhibit ECM-like properties.

**Particulate ECM**

In one embodiment of the invention the hydrogel layer comprises particulate ECM as an ECM-mimicking structure.

The extracellular matrix (ECM), as understood in the context of the present application, is the non-cellular portion or material of animal or human tissues that surrounds cells. The ECM material may be obtained from any kind of tissue by the use of physical, enzymatic, and/or chemical methods well known in the art. In principal, the major components of ECM comprise fibrous elements (particularly collagen, chitosan, elastin or reticulin), link proteins (for example fibronectin or laminin) and space-filling molecules (for example glycosaminoglycans). Moreover, ECM typically includes growth factors, cytokines, and other biological molecules that are known to attract cells and to promote cellular proliferation.

For preparing the particulate ECM the skilled person can draw on a broad spectrum of potential ECM sources. In one embodiment the ECM can be isolated from *in vitro* cultivated cells. Preferably cells include cultured chondrocytes, fibroblasts, keratinocytes or epithelial cells.

The ECM material for preparing the particulate ECM used in the present invention may be obtained from tissue harvested from any animal, but in particular from animals raised for meat production, including but not limited to, pigs, cattle and sheep. Using these animals as sources for the ECM material reduces the costs of the material. Other warm-blooded vertebrates may also hold as a source of tissue. Moreover, the ECM material may also be obtained from tissue of human corpses. The ECM material can be obtained from any organ, but is preferably obtained from intestinal tissue, bladders, liver, spleen, stomach, lymph nodes or skin. Preferably, the ECM material used in the present invention is derived from human cadaver skin, porcine urinary bladder submucosa (UBS), porcine urinary bladder matrix (UBM), or porcine small intestinal submucosa (SIS). When human tissue is used as the source of the ECM material, the risk of an immunogenic response or immune rejection can be reduced. However, using ECM material of animal origin may reduce the risk of a transfer of diseases. In a preferred embodiment of the present invention, the ECM material is of animal origin. Particularly preferred is an ECM material derived from warm-blooded mammals, due to their similarity to humans. Preferably, the ECM material used in the present invention is of porcine origin. Most preferred is an ECM material derived from Urinary Bladder Matrix (UBM) or small intestine submucosa (SIS) of porcine origin.

In a preferred embodiment of the invention the particulate ECM is preferably a decellularized ECM. Hereby, the isolated tissue is treated with a series of oxidizing agents that remove the cellular and nuclear material from the isolated tissue while substantially retaining the biological and mechanical properties, as well as the biochemical composition of the resulting ECM.

Decellularizing agents that can be used in the methods of the invention are those agents that are effective in removing cellular and nuclear material from the isolated tissue without substantially compromising the biocompatible, biological and mechanical properties or the biochemical composition of the ECM. Examples of decellularizing agents that can be used for decellularization of the tissue include, but are not limited to, oxidizing agents (e.g., hydrogen peroxide, peroxy acids), ascorbic acid (vitamin C), chelating agents (e.g., EDTA, EGTA), methionine, cysteine, maleic acid, and polymers that bind to DNA (e.g., Poly-L-lysine, polyethylimine (PEI) and polyamidoamine (PAMAM)).

In one embodiment of the invention, the particulate ECM has a mean diameter of between 1 $\mu$m and 2000 $\mu$m, preferably of between 10 $\mu$m and 1000 $\mu$m, more preferably of between 50 $\mu$m and 250 $\mu$m and especially of approx. 150 $\mu$m. This is determined by a Mastersizer 200 from Malvern Instrument for volume weighted mean. For example, a surface weighted mean of 100 $\mu$m, can have the smallest particles of 3 $\mu$m and the largest particles of 750 $\mu$m. A volume weighted mean would in this case be 250 $\mu$m.

**Collagen protein structure**

In one embodiment of the invention the hydrogel layer comprises a collagen protein structure as an ECM-mimicking structure.

In a preferred embodiment of the invention the collagen protein structure is selected from the group consisting of types I, II and III collagens and mixtures thereof.

Soluble collagens of the types I, II and III collagen are prepared by limited enzymatic digestion of tissue enriched in such types of collagen and subsequent formation of a collagen-based solution (i.e. a soluble collagen dissolved in a suitable solvent, such as dilute hydrochloric acid, diluted acetic acid or the like).

Insoluble collagens can be obtained from the following typical sources: type I collagen: bovine, chicken and fish skin, bovine and chicken tendons and bovine and chicken bones including fetal tissues; type II collagen: bovine articular cartilage, nasal septum, sternal cartilage; and type III collagen: bovine and human aorta and skin.

In another embodiment of the invention the collagen protein structure as used for the polymer layer is a human recombinant collagen.

The term "human recombinant collagen" refers to collagen manufactured by culturing a non-human organism genetically modified to express at least one human gene encoding a collagen. The human recombinant collagen is suitably selected from the group consisting of collagen type I, type II, type III, type IV, type V, type VI, type VII, type VIII, type IX, type X, type XI, type XII, type XIII, type XIV, type XV, type XVI, type XVII, type XVIII, type XIX, type XX, type XXI, type XXII, type XXIII, type XXIV, type XXV, type XXVI, and type XXVII. The collagen can be collagen of one type free of any other type, or can be a mixture of different types of collagen. Suitably, the collagen comprises or consists essentially of collagens selected from the group consisting of type I collagen, type III collagen and mixtures thereof.

Human recombinant collagen may be provided by any suitable method known in the art. For example, the step of providing human recombinant collagen may comprise the following protocol described in U. S. Pat. No. 5, 962, 648, the entire content of which is incorporated herein by reference. Further recombinant processes are set forth in U.S. Pat. No. 5,593,859 and WO2004/078120, which are also incorporated herein by reference. Preferably, collagen is produced by culturing a cell which has been genetically modified to express at least one gene encoding the polypeptide comprising collagen and additional genes encoding the subunits of the post-translational modifying enzyme prolyl 4-hydroxylase, followed by the purification of the resultant collagen monomer therefrom. The recombinant collagen solution may be subsequently subjected to polymerization or cross-linking conditions to produce insoluble fibrous collagen.

Bovine collagen is a mixture of collagen type I (85%) and collagen type III (15%), a combination which is set by nature and cannot be varied. An advantage of recombinant collagen is that collagen type I and collagen type III are made independently of one another, and so any combination of type I and type III collagen can be made. The products according to the present invention may suitably comprise human collagen type I and human collagen type III in any ratio. For example, the products may comprise human collagen type I and human collagen type III in a ratio by weight of 100:0, 80:20, 60:40, 50:50, 40:60, 20:80 or 0:100, or anywhere in-between. Preferably, the ratio by weight of human collagen type I:human collagen type III is greater than about 50:50, and preferably it is greater than about 70:30, for example about 80:20. Suitably, the type I human recombinant collagen makes up at least about 75% by weight of the total human recombinant collagens in the material.

**Collagen-like protein structure**

In one embodiment of the invention the ECM-mimicking structure is a collagen-like protein structure. In the context of the present invention the "collagen-like protein structure" is defined as a recombinant collagen-like protein of bacterial origin.

Typically, it represents a modular collagen-like protein that is stable at mammalian body temperature either in its native state or after chemical cross-linking and aggregates to form fibrils.

In a preferred embodiment the collagen-like protein structure of the invention is defined by the distinctive supercoiled triple-helix conformation, having a $(Gly\text{-}Xaa\text{-}Yaa)_n$ amino acid sequence. In this configuration, Gly provides a glycine residue with Xaa and Yaa independently comprising any known imino or amino acid residue for each repeat unit. Unique properties of the collagen triple-helix motif include its molecular hydrodynamic properties, extensive hydration, ability to bind diverse ligands, and capacity to self-associate to form fibrils and other higher order structures. These distinctive features have been exploited by nature to fill a wide range of structural and functional niches.

In a more preferred embodiment, the collagen-like protein structure comprises the formula I:

$$(I) \qquad [(Gly\text{-}Xaa\text{-}Yaa)_m\text{-}(insert)_n]_p$$

where m is between 1 to 200 and $(Gly\text{-}Xaa\text{-}Yaa)_m$ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site.

The overall content of Xaa and Yaa provides a proline rich structure where the total percentage of proline of all residues in the Xaa and Yaa positions is greater than 19%, but optimally, though not exclusively, between 19.5-40%. Alternatively, or in conjunction with the proline concentration, the triple helical motif may also contain a concentration of charged residues (e.g. Asp, Glu, Lys, Arg, His) of greater than 14% and optimally, though not exclusively, between 14-35%. Such domains should also aggregate, either naturally or synthetically, at a neutral pH or otherwise using one or a variation of such protocols discussed herein or otherwise known in the art.

The triple helical domains may be isolated from one or multiple pathogenic or non-pathogenic bacterial organisms. By way of example, the triple helical domains can include domains derived from *Streptococcus pyogenes, Clostridium perfringens, Methylobacterium sp.4-46, Solibacter usitatus* Ellin6076 or *Rhodopseudomonas palustris* tie-1, which exhibit the desired heat stability in either its native state or after stabilization by chemical cross-linking. Such sequences may include those identified in U.S. Patent No. 6,953,839 or WO2010/091251, the contents of which are incorporated herein by reference. Alternatively, each triple helical domain may include repeats, fragments, homologues or combinations of the foregoing peptide sequences.

Also important to the stabilizing structure of the collagen-like products of the instant invention is the proper formation of the triple helical structure. While some triple helical domains are able to be formed using the native mechanisms of the expression vehicle, proper folding of the recombinant bacterial collagen-like structure represented by formula I may also be assisted using globular or variable (V) domains. Such globular domains may be expressed directly or indirectly to the N-terminus and/or the C-terminus of the triple helical domain and can facilitate both post-translational folding and refolding following heat denaturation. One nonlimiting example of an N-terminus domain is the entirety of or a portion of a variable (V) domain, the Scl2 sequence found in *Streptococcus pyogenes,* which may be provided as the following peptide sequence:

EENEKVREQEKLIQQLSEKLVEINDLQTLNGDKESIQSLVDYLTRRGKLEEEWME

YLNS GIQRKLFV (SEQ ID NO: 42).

A non-limiting example of a C-terminus variable domain or V domain may be isolated from the organism *Rhodopseudomonas palustris* tie-1, which may be provided as the following peptide sequence:

PSIEQVMPWLHLIFDAYEDYKAQRAREARELEERLAAEALEQAAREAAEREV

AAAIEAANAEAEIMLDDETHAEGGKKKKKRKHKD (SEQ ID NO: 43)

Further stabilizing structures such as "coiled coil forming sequences" or "foldons" may include those identified in WO2010/091251, the contents of which are incorporated herein by reference.

**Peptide**

In an embodiment of the invention, the hydrogel comprises a peptide comprising an MMP-cleavage site. In a preferred embodiment of the invention said peptide is part of a covalent polymer-peptide conjugate.

In a preferred embodiment of the invention, heparin is bound covalently to polyethylene glycol via short enzyme-cleavable peptide sequences as crosslinking molecule, which preferably cleavage-sites for matrix metalloproteinases (MMP).

In one embodiment of the invention, the functionalization of heparin with enzyme-cleavable peptide sequences takes place by reaction of the carboxyl groups of heparin activated with 1-ethyl-3-(3-dimethylaminopropyl) carbodiimides/N-hydroxysulfosuccinimide (EDC/s-NHS) with the amino group of the N-terminus of the peptide, and then gel formation takes place by reaction of heparin functionalized with the peptides, by reaction of the carboxyl group on the C-terminus of the peptide activated with 1-ethyl-3-(3-dimethylamillopropyl) carbodiimides/N-hydroxysulfosuccinimide (EDC/ s-NHS), with amino groups of functionalized PEG.

In an embodiment of the invention, the enzyme cleavage site is given by one of the following amino acid sequences as given in single letter code:

- GPQG↓IAGQ (SEQ ID N0: 3),
- GPQG↓IWGQ (SEQ ID N0: 4),
- GNVG↓LAGA (SEQ ID N0: 5),
- GPQG↓IA (SEQ ID N0: 6),
- GPQG↓IL (SEQ ID N0: 7),
- GPQG↓LA (SEQ ID N0: 8),
- GPQG↓LL (SEQ ID N0: 9),
- GPLG↓IA (SEQ ID N0: 10),
- GPLG↓IL (SEQ ID N0: 11),
- GPLG↓LA (SEQ ID N0: 12),
- GPLG↓LL (SEQ ID N0: 13),
- GPRG↓LQ (SEQ ID N0: 14),
- GPTG↓LA (SEQ ID N0: 15),
- GPQGIAGQRGVVGLP (SEQ ID NO: 16)
- GPQGLLGAPGILGLP (SEQ ID NO: 17)
- GEQGPQGLP (SEQ ID NO: 18)
- GPQGLAGQRGIV (SEQ ID NO: 19)
- GAQGPPGAPGPLGIAGITGARGLAGPPGMPGPRGS (SEQ ID NO: 20)
- GPLGIAGITGAR (SEQ ID NO: 21)
- GAQGPPGAPGPLGIAGITGARGLA (SEQ ID NO: 22)
- GPSGAEGPPGPQGLAGQRGIVGLPGQRGERGFP (SEQ ID NO: 23)
- GPQGLAGQRGIV (SEQ ID NO: 24)
- GPSGAEGPPGPQGLAGQRGIVGLP (SEQ ID NO: 25)

The advantage of this is the synthesis of a network of star-PEG and heparin joined together covalently, which can be degraded locally by cleavage of the peptide bridges by the enzyme activity of individual cells or by the activity of MMPs, which are released during wound-healing. As a result, a partial restructuring and replacement of the synthetic matrix with material secreted by the cells is possible, moreover it is possible for the cells to migrate into the material and unite, to form therapeutically important structures. Regardless of the type of crosslinking - either directly between heparin and star-PEG or via the cleavable peptide sequences-the physical properties of the scaffold can be varied over a wide range. The cell adhesion of the materials can be controlled by the targeted modification of heparin with short peptide sequences, for example by integrin-binding arginine-glycine-aspartic acid (RGD) sequences.

In one embodiment of the invention, the covalent polymer-peptide conjugate comprises conjugates of two or more peptides which are coupled to a polymer chain. The peptide sequence contains a repetitive dipeptide motif $(BA)_n$, wherein B is an amino acid with a positively charged side chain, A is alanine and n is a number from 4 to 20, indicating the number of individual repeating dipeptide modules (BA) within the dipeptide motif $(BA)_n$. The amino acid B is preferably arginine, with the one-letter code R, or lysine, indicated by the one-letter code K. Except for the L- and D-variants of arginine and lysine as natural amino acids, moreover generally all non-natural amino acids which are positively charged (basic) are suitable as amino acid B. The repetitive dipeptide motif represents a minimal heparin-binding motif which enables the formation of a non-covalent hydrogel matrix with self-organizing properties. Preferred repetitive dipeptide motif (BA)$_n$ are disclosed in WO2014/040591, the contents of which are incorporated herein by reference.

Alterations of this repetitive dipeptide motif can result in changes of the properties of the hydrogel. In principle, even a hydrogel without oligosaccharide is achievable. The flexible design of the peptide sequence can result in a wide variety of gel properties like gelation state, gelation rate and protein binding properties, but also properties such as but not limited to light sensitivity or enzyme-mediated biodegradability.

According to another embodiment of the invention, the hydrogel further comprises a light-cleavable chemical linker between the polymer chain and the peptide sequence containing the dipeptide motif repetitive (BA)$_n$.

In another embodiment of the invention, the hydrogel further comprises a pH-sensitive chemical linker between the polymer chain and the peptide sequence containing the dipeptide motif repetitive (BA)$_n$.

According to another embodiment of the invention, the hydrogel further comprises an enzyme cleavable linker between the polymer chain and the peptide sequence containing the dipeptide motif repetitive (BA)$_n$. As enzymatically cleavable linker, the hydrogel preferably comprises a peptide sequence which is a proteolytically active substrate, more preferably a proteolytically active substrate for MMP.

In another embodiment of the invention, the hydrogel comprises an oligonucleotide sequence as an enzymatically cleavable linker, which is a substrate for nucleases.

In an embodiment of the invention, the hydrogel comprises a heparin modified with an adhesion protein of the sequence cycloRGDyK.

In an advantageous further development of the invention, the hydrogel is loaded with the growth factors basic fibroblast growth factor b-FGF and vascular endothelial growth factor VEGF, wherein the hydrogels are incubated with a solution of b-FGF or VEGF with a concentration of 1-5 $\mu$g/ml.

In certain embodiments the hydrogel comprises a micro particles patterned within the hydrogel. In one preferred embodiment, growth factors are provided within micro particles (e.g., PLGA micro particles) entrapped within a hydrogel (e.g., PEG hydrogel) base. Such systems have been constructed to deliver agents with two different delivery profiles (see, e.g., Wang et al. (2011) Pharmaceutical Res., 28(6): 1406-1414).

In various embodiments the growth factor(s) are released slowly and sustained over at least one day, or over at least 3 days or over at least 5 days, or over at least one week, or over at least two weeks, or over at least 3 weeks, or over at least one month, or aver at least two months, or aver at least three months, or over at least six months. Typically the growth factor(s) are released directly to the target tissue in the body.

In an embodiment of the invention, the hydrogel comprises silver nanoparticles, incorporated in anticoagulant poly(ethylene glycol) (PEG)-heparin hydrogel coatings on thermoplastic polyurethane materials. For prolonged antimicrobial activity, the silver-containing starPEG-heparin hydrogel layers were shielded with silver-free hydrogel layers of otherwise similar composition

In a preferred embodiment of the invention the peptide comprising a MMP cleavage site or the collagen like protein structure comprises one or more of the following:

 a. a heparin binding dipeptide motif (BA)$_n$, wherein B is an amino acid with a positively charged side chain, A is alanine an n is an integer between 4 and 20;
 b. a pH-labile chemical linker which is preferably to the C-terminally or N-terminally attached to said peptide;
 c. an integrin binding site, being preferably an RGD motif or having an amino acid sequence of GFPGER (SEQ ID No. 26) or GEFYFDLRLK (SEQ ID NO. 27);
 d. a photo-cleavable linker which is preferably to the C-terminally or N-terminally attached to said peptide;
 e. an IKVAV (SEQ ID NO. 28) motif;
 f. a fibronectin binding domain, having preferably the amino acid sequence of GLAGQRGIVGLPGQRGER (SEQ ID No. 29);
 g. a Discoidin domain-containing receptor 2 (DDR2) domain, having preferably an amino acid sequence of GPRGQPGVMGFP (SEQ ID No. 30);
 h. a non-collagen-natural break having a peptide sequence spaced between two glycine residues of the insert sequence of the collagen-like protein structure; having an amino acid sequence of SEQ ID No. 31 to 37;
 i. a heparin binding domain;
 j. an $\alpha$1$\beta$-integrin binding domain, being preferably GLPGER, GFPGER or GFPGEN (SEQ ID Nos. 38-40)
 k. an ECM retaining moiety comprising the amino acid sequence DHLSDNYTLDHDRAIH (SEQ ID No. 41).

The peptide might contain insert sequences to improve the bendability or elasticity of the biomaterial or otherwise serve as a natural binding domain or biological cleavage sequence. Natural breaks or interruption sequences, for example, may include those of non-fibrillary human collagens, which are typically provided as 1 to 50 amino acids spaced between two glycine residues. While the instant invention is not so limited, examples of such sequences include those provided as follows (SEQ ID Nos. 31 to 37) as well as repeats, fragments, homologues or combinations thereof:

- GAAVMG (SEQ ID No. 31),
- GDSAVILG (SEQ ID No. 32),
- GDMVVSRVKG (SEQ ID No. 33),
- GRLVDTGPGAREKG (SEQ ID No. 34),
- GSVPNVDRLLETAGIKASALREIVETWDESSGSFLPVPERRRG (SEQ ID No. 35),
- GPGEFYFDLRLKGPG (SEQ ID No. 36),
- GQISEQKRPIDVEFQK (SEQ ID No. 37).

In one embodiment of the invention particulate ECM, collagen protein structure, collagen-like protein structure and peptide comprising a matrix metalloproteinase (MMP) cleavage site is not bound to the polymer and/or highly negative oligo- or polysaccharide of the hydrogel but is separated within the hydrogel.

In a more preferred embodiment of the invention particulate ECM, collagen protein structure, collagen-like protein structure and peptide comprising a matrix metalloproteinase (MMP) cleavage site is bound in a covalent or non-covalent form to the polymer and/or highly negative oligo- or polysaccharide of the hydrogel.

**Further characteristics of the wound care product**

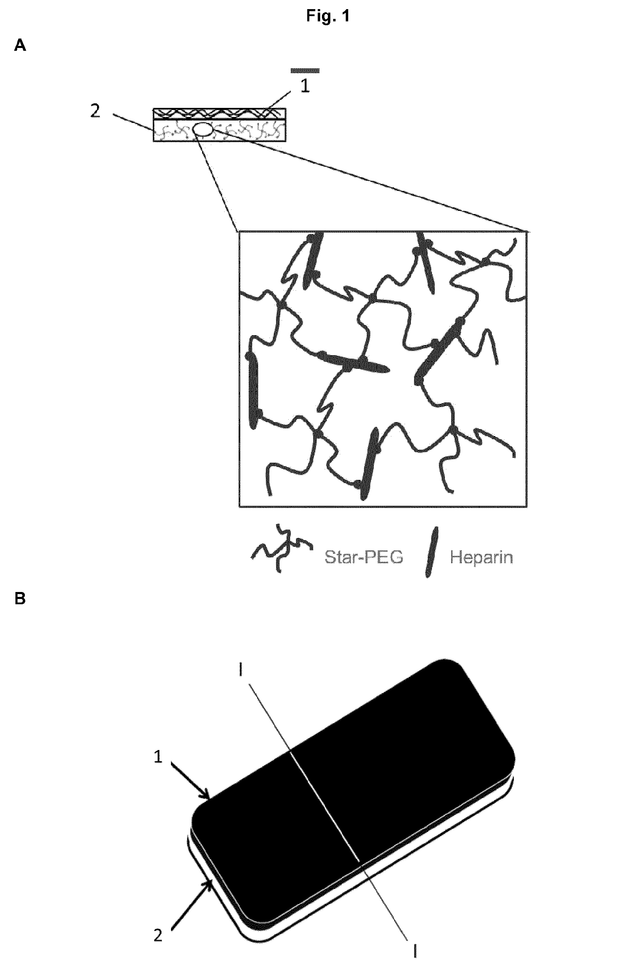

The dimension of the surface area of the wound care product may be selected according to the size of the wound. Typically, a wound care product of the present invention may have a surface area of from 5 $cm^2$ to 400$cm^2$, and particularly of from 25 $cm^2$ to 200$cm^2$, such as 100$cm^2$. Also the shape of the wound care product according to the present invention may vary with respect to the wound to be treated, and the present invention encompasses, for example, rectangular, square-, circle- or oval-shaped wound care products. For example, the wound care product may have a rectangular shape with rounded corners as depicted in Fig. 1 B.

The wound care product of the present invention contains at least two layers, a nanocellulose layer and a separate and distinct hydrogel layer.

Typically, for obtaining a wound care product according to the present invention, a hydrogel coating has been applied to the nanocellulose layer.

The nanocellulose layer of the invention is preferably directly attached to the hydrogel layer.

In a further embodiment, the nanocellulose layer and the hydrogel layer may be separated by an additional intermediate layer that is located between both layers. According to this embodiment of the wound care product, there is no direct contact between the nanocellulose layer and the hydrogel layer. The advantage of this embodiment could be an enhanced strength of the multilayer or a control of the passage of cells or other biological molecules between the two layers. Further, this additional intermediate layer may be used to ensure the attachment of both layers to the product. The additional intermediate layer (IL) is designed and composed according to prior art, and may comprise, for example, a material based on polysaccharide, animal- or plant protein, animal connective tissue, fibrin, silicone, epoxide, acrylate, cyano-acrylate, (poly)urethane, enzymes (such as transglutaminase), L-DOPA or also gelatin-resorcinol-formaldehyde-glutaraldehyde ("French glue"), and may have a thickness of from 1 $\mu$m to 500 $\mu$m, and particularly of from 3 $\mu$m to 150 $\mu$m. It shall be understood that such an additional intermediate layer or separating layer may be provided between any layers in any of the embodiments described herein, even if not explicitly mentioned in the context of the other embodiments.

It shall be understood that the wound care product according to the present invention may include still further layers of nanocellulose and further hydrogel layers. Preferably, the wound care product of this embodiment includes alternating polymer and ECM layers.

In one embodiment the multilayer of the invention can be provided with additional layers such as, e.g., a covering layer.

In a preferred embodiment the covering layer is a polyurethane film having preferably a thickness between 10 and 50 $\mu$m, preferably between 15 and 40 $\mu$m and more preferably between 18 and 30 $\mu$m.

The PU film is used to control water steam permeation and/or to control the fluid handling capacity. Thereby, this additional layer is useful to develop a wound care product that is useful for different exuding levels.

In a preferred embodiment of the invention the multi-layered wound care product has a moisture vapor transmission rate (MVTR) as measured with the upright MVTR set-up according to DIN ISO 13726-2 of more than 500 g/$m^2$/24h at 21 °C $\pm$ 2 °C and 60 % $\pm$ 15 % relative humidity, preferably of more than 750 g/$m^2$/24h, and preferably of more than 1000 g/$m^2$/24h.

In a special embodiment of the invention the multi-layered wound product exhibits one of the following product layer characteristics:

- PEG hydrogel with covalently bound RGD sequence, attached to a layer of bacterial nanocellulose

- Star-PEG-heparin hydrogel with incorporated particulate ECM, attached to a layer of bacterial nanocellulose

- Chitosan hydrogel with incorporated particulate ECM, attached to a layer of bacterial nanocellulose

- Degradable PEG-dextran hydrogel with incorporated particulate ECM, attached to a layer of bacterial nanocellulose

- Star-PEG hydrogel with incorporated particulate ECM and growth factors, attached to a layer of bacterial nanocellulose

- Degradable star-PEG hydrogel with incorporated biomimetic sequence (e.g. RGD), attached to a layer of bacterial nanocellulose

- Degradable star-PEG-heparin hydrogel with incorporated particulate ECM attached to a layer of bacterial nanocellulose

**Use of the wound-care product**

In a preferred embodiment of the invention the hydrogel layer represents the bottom layer of the multilayer of the invention and therefore is in contact to the wound, which is preferably located on the skin.

The multi-layered wound care product of the invention is particularly useful in the treatment of acute wounds, burn wounds, chronic wounds, and/or surgical wounds. The wound care product of the present invention may further be used in plastic surgery as well as for tissue engineering.

As such it can be used for the treatment of burns, partial and full-thickness wounds, pressure ulcers, venous ulcers, arterial ulcers, diabetic ulcers, chronic vascular ulcers, draining wounds, tunneled or undermined wounds, surgical wounds such as donor sites/grafts, post-Mohs surgery, post-laser surgery, podiatric dehiscence, and wound dehiscence, trauma wounds such as abrasions, lacerations, first, second, or third degree burns, and skin tears.

In the field of tissue engineering the wound care product of the invention can be used for remodelling of soft tissue, bone, cartilage, ligaments and tendons or dental applications.

For the medical use, it is required that the wound care product of the invention is provided in sterile form. This can be achieved by packaging the sterile product in a bacterial tight material with a marking on the packing that the product is sterilized. Bacterial tight materials are well known to the person skilled in art.

For preparing the polymer layer of multi-layered wound care of the invention the skilled person can use the various manufacturing methods as established for hydrogels and for nanocellulose layers.

In a further aspect the invention provides a method of preparing a multilayered wound care product comprising the following steps:

a. Producing the bio-nanocellulose:

i. cultivation of bacteria recombinantly expressing and excreting biocellulose
ii. a depyrogenisation step of the produced biocellulose
iii. several washing- and neutralizing steps
iv. a drying step to adjust the water content of the product (mechanical, freeze-drying or the like)

b. Producing the hydrogel layer:

i. Optional: functionalizing the hydrogel monomer units with (bio-) functional domains
ii. Optional: addition of an aqueous solution of particulate ECM
iii. Preparation of the hydrogel and crosslinking of the monomer units
iv. Several washing steps

c. Producing the final wound care product

i. The product as result from step (a) and the product as resulting from step (b) are combined to produce the final wound care product.

**Examples:**

**1. Preparation of a star-PEG hydrogel with covalently bound RGD sequence, attached to a layer of bacterial nanocellulose**

The preparation of the peptide-functionalized hydrogel was performed essentially based on the method as disclosed by Fittkau et al. 2005 (Biomaterials 26: 167-174). The bacterial nanocellulose layer can be prepared as taught by Jung et al., 2010 (Appl Biochem Biotechnol 162: 486-497).

a. <u>PEG acrylation</u>: A 10m% 4-arm PEG solution in toluene is dried by azeotropic distillation and diluted back to original concentration through anhydrous addition of dichloromethane (DCM). After cooling in an ice bath and addition of 50% molar excess of triethylamine (TEA), acryloyl chloride (AcCl) (50% molar excess) is added dropwise over 5 min. Ice bath is removed and the reaction continued under an Argon atmosphere at room temperature (RT) for 24 h. Filtering, precipitation, extraction from an aqueous solution (10% PEG-4Ac, 0.5% NaCl, pH=6) into DCM, final precipitation (hexane), and drying (in vacuo, 24 h, RT)
b. RGD Peptide: the peptide 'GCGGGRGDSPG' is synthesized via solid state chemistry and purified subsequently
c. PEG-4Ac (50% of final volume) and peptide aliquots (25 % of final volume) are mixed and incubated for 1 h at room 37 °C. Peptide concentration thereby ranges between 0.1 mol% and 40 mol%.
d. PEG-2SH (25% of final volume) is added to the solution from step c., mixed well and incubated in a sterile mould for 1 h at 37 °C to allow for gel polymerization
e. The hydrogel prepared in step d. is connected to a layer of BNC by standard technologies known to the art, such as 'biological glue' (e.g. fibring glue) as mentioned above, or by means of standard chemical binding reactions.

**2. star-PEG-heparin hydrogel with incorporated particulate ECM, attached to a layer of bacterial nanocellulose**

The preparation of the star-PEG-heparin hydrogel was performed essentially based on the method as disclosed by Freudenberg et al. 2009 (Biomaterials 30: 5049-5060).

a. 1-Ethyl-3-(3-dimethylaminopropyl)carbodiimid / sulfo-N-hydroxysuccinimide (EDC/sulfo-NHS 2:1 ratio) and heparin (MW 14000 Da) are dissolved in decarbonized water and incubated for 15 minutes at 2-4 °C
b. Amine end-functionalized 4-arm PEG (MW 10000 Da) is dissolved in ddH$_2$O and added to solution prepared in step a. The molar ratio of heparin to star-PEG is chosen to be 0.5. An aqueous solution with the desired concentration of particulate ECM is added to this solution.
c. Mix for 15 minutes at 8 °C and keep gels at room temperature for 15 hours.
d. Removal of excessive EDC/sulfo-NHS and star-PEG/heparin by several washing and swelling steps
e. Gel formation by adding an appropriate amount of the solution prepared in 2.d. onto a layer of BNC coated with an additional layer of fibrin glue and incubated for 14 h at 37 °C to allow for gel polymerization.

**3. Preparation of a chitosan hydrogel with incorporated particulate ECM, attached to a layer of bacterial nanocellulose**

a. Powdered chitosan (2.8 % w/v; MW 250 kDa) is dissolved in aqueous 2 % w/v acetic acid;
b. An aqueous solution with the desired concentration of particulate ECM is added to this solution in a ratio of 1:1;
c. Variation 1: The resulting solution is stirred over night at room temperature and afterwards casted into a sterile mould. The transparent chitosan/ECM film obtained after a drying step at 60 °C for 24 hours is removed from the mould - this results in a dry non elastic chitosan ECM film;
d. Variation 2: To solution as resulting from step b. 2-10% Glycerin or PEG 200-1000 is added stirred over night at room temperature and afterwards casted into a sterile mould. The transparent chitosan/ECM film obtained after a drying step at 60 °C for 24 hours is removed from the mould - this results in a dry elastic chitosan ECM film;
e. Variation 3: The solution as resulting from step b. is stirred over night at room temperature and afterwards cool down to 4-8°C. In the solution a 1% glutaraldehyde (or another cross linking agent) solution is added. The solution is mixed fast and very well. The solution is placed in a mould (e.g. Polytetrafluorethylene) and heated up for 0.5-2h to 60°C. During heating a transparent hydrogel is formed. This results in a crosslinked hydrogel chitosan ECM film.
f. A layer of BNC is connected to a layer of BNC by standard technologies known in the art, such as 'biological glue' (e.g. fibrin glue) as mentioned above, or by means of standard chemical binding reactions.

**4. Preparation of a degradable PEG-dextran hydrogel with incorporated particulate ECM, attached to a layer of bacterial nanocellulose**

The preparation of the PEG-dextran hydrogel was performed essentially based on the method as disclosed by Sun et al. 2011 (PNAS 108(52): 20976-20981).

 a. Dextran-Allyl Isocyanate-Ethylamine (Dex-AE) synthesis: dextran is dissolved in anhydrous dimethyl sulfoxide (e.g. 2 g in 20 mL) at room temperature under nitrogen gas. Reaction catalyst Dibutyltin dilaurate (0.210 ml) and allyl Isocyanate (0.24 ml) are added to the reaction and incubated for 6 hours at 35 °C
 b. The solution from step a. is precipitated in cold excess isopropanol, filtered, and dialyzed against ddH$_2$O for 3 days and subsequently lyophilized for additional 3 days.
 c. The product from step b. is dissolved in dimethyl sulfoxide (e.g. 2 g in 30 ml) under nitrogen gas.
 d. 2-bromoethylamine hydrobromide (3.75 g) is dissolved in dimethyl sulfoxide (10 ml).
 e. The catalyst TEA (11.2 ml) is added to the solution from step c.;
 f. The solution from step d. is added to the solution from step e. dropwise;
 g. The reaction is allowed to run for 6 hours at 50 °C;
 h. The triethylamine salt is filtered and the resulting solution precipitated in cold excess isopropanol and also filtered;
 i. The product from step h. is dialyzed against ddH$_2$O for 3 days and lyophilized for additional 3 days;
 j. Dex-AE/PEGDA (polyethylene glycol diacrylate) Hydrogels: Dex-AE and PEGDA are dissolved in a weight ratio of 80:20 in phosphate buffered saline containing 0.1 % (w/w) 2-methyl-1-[4-(hydroxyethoxy)phenyl]-2-methyl-1-propanone (Irgacure® 2959). An aqueous solution with the desired concentration of particulate ECM is added to this solution. This mixture is connected to a layer of BNC by standard technologies known to the art, such as 'biological glue' (e.g. fibrin glue) as mentioned above, or by means of standard chemical binding reactions;
 k. The hydrogel is crosslinked for 10 minutes with 10 mW/cm$^2$ with UV light.

**5. Preparation of a star-PEG hydrogel with incorporated particulate ECM and growth factors, attached to a layer of bacterial nanocellulose**

The preparation of the PEG/RGD peptide hybrid hydrogel was performed essentially based on the method as disclosed by Liu et al. 2010 (Macromol Rapid Comm 31: 1148-1154).

 a. tetra-PEG-acrylate is dissolved in phosphate buffered saline (3.75 mg, 0.357 μmol)
 b. The growth factors FGF and VEGF are added to this solution with a final concentrations of 3 μg/ml
 c. Particulate ECM is added at the desired concentration to this solution and dissolved.
 d. PEG dithiol solution (1.4 mg, 0.69 μmol) is added as crosslinker
 e. Final precursor solution is chosen to be 10 % w/v
 f. Hydrogels are formed by incubation at 37 °C
 g. The products from 5.f. are connected to a layer of BNC by standard technologies known to the art, such as 'biological glue' (e.g. fibrin glue) as mentioned above, or by means of standard chemical binding reactions.
 h. Incubation at room temperature for 12 hours.

**6. Preparation of a degradable star-PEG hydrogel with incorporated RGD sequence, attached to a layer of bacterial nanocellulose**

The preparation of the star-PEG hydrogel was performed essentially based on the method as disclosed by Ali et al. 2013 (BioResearch Open Access 2(4): 241-249).

 a. Synthesis of RGD-PEG: RGDS peptide (obtained via solid state synthesis) is dissolved in anhydrous dimethyl sulfoxide with 2 μl diisopropylethylamine and then added dropwise to dry acryolyl-4 arm PEG-succinimidyl carboxymethyl at a molar ration of 1 : 1.2;
 b. The product is dialyzed against water with a 5000 Da molecular cut off membrane and lyophilized;
 c. Synthesis of the protease-sensitive peptide sequence bound to PEG: GGGPQGIWGQGK peptide (obtained via solid state synthesis) is dissolved in anhydrous dimethyl sulfoxide with 2 μl diisopropylethylamine and then added dropwise to dry acryolyl-4 arm PEG-succinimidyl carboxymethyl at a molar ration of 2 : 1;
 d. The product is dialyzed against water with a 5000 Da molecular cut off membrane and lyophilized;
 e. Formation of hydrogels: mixing of product from 6.c. (10 % w/v) in sterile HEPES-buffered saline with 1.5 % triethanolamine and 3.95 μl N-vinyl-2-pyrrolidone (NVP);
 f. Addition of 5 mM RGD-PEG (product from 6.a.) to the solution in 6.e. and addition of this mixture onto a layer of BNC;

g. Hydrogel and BNC layer are connected by standard technologies known to the art, such as 'biological glue' (e.g. fibrin glue) as mentioned above, or by means of standard chemical binding reactions;
h. Cross-linking of hydrogels with white light for 35 seconds

**7. Preparation of a degradable star-PEG-heparin hydrogel with incorporated particulate ECM attached to a layer of bacterial nanocellulose**

The preparation of the star-PEG hydrogel was performed essentially based on the method as disclosed by Maitz et al. 2013 (Nature Communications 4: Article 2168).

a. Synthesis of star-PEG-peptide conjugate: the peptide GPQGLLGAPGILGLP (SEQ ID NO: 17, obtained via solid state synthesis) is dissolved phosphate buffered saline and added to a solution of maleimide-functionalized 4-arm PEG. A small amount of tris(2-carboxyethyl)phosphine chloride is added to the solution. After overnight incubation the solution is dialyzed against water (molecular weight cut off 5000 da);
b. 1-Ethyl-3-(3-dimethylaminopropyl)carbodiimid / sulfo-N-hydroxysuccinimide (EDC/sulfo-NHS 2:1 ratio) and heparin (MW 14000 Da) are dissolved at stoichiometric balanced concentrations in decarbonized water and incubated for 15 minutes at 2-4 °C;
c. Removal of excessive EDC/sulfo-NHS and star-PEG-peptide/heparin by several washing and swelling steps with phosphate buffered saline;
d. The product from 7.a. is added to the solution in step b. at a molar ratio of 1:1, mixed at 8 °C for 15 minutes with 0.1 % (w/w) 2-methyl-1-[4-(hydroxyethoxy)phenyl]-2-methyl-1-propanone (Irgacure® 2959). An aqueous solution with the desired concentration of particulate ECM is added to this solution. This mixture is added onto a layer of BNC and crosslinked for 10 minutes with 10 mW/cm$^2$ with UV light;
e. Hydrogel and BNC layer are connected by standard technologies known to the art, such as 'biological glue' (e.g. fibrin glue) as mentioned above, or by means of standard chemical binding reactions.

**Brief description of the drawings:**

These and other aspects of the invention will be apparent from and elucidated with reference to the embodiments described hereinafter.
The invention will now be described, by way of example, based on embodiments with reference to the accompanying drawings.
In the drawings:

Fig. 1 shows a principal sketch of the wound care product according to a first embodiment in cross section **(A)** or in a perspective view **(B).**

In the Figures, like numbers refer to like objects throughout. Objects in the Figures are not necessarily drawn to scale.

**Detailed description of embodiments:**

Various embodiments of the invention will now be described by means of the Figures.
Fig. 1 shows a principal sketch of the wound care product according to a first embodiment in cross section **(A)** or in a perspective view **(B).** The wound care product consists of a nanocellulose layer (1) and a hydrogel layer (2). The hydrogel consists of four arm starPEG which is terminally linked to a heparin-binding peptide, whereby the peptide comprises a MMP cleavage site. When mixing the peptide-functionalized starPEG and the heparin a starPEG-heparin hydrogels scaffold is formed *in situ.* The wound care product may be applied as such to a skin wound. To this, the wound care product has a surface that will face the wound when being applied and will come in direct contact with the wound, and has an opposite surface that will face away from the wound and will thus initially not contact the wound when applied. In the embodiment depicted in Fig. 1 B, the lower layer provides the surface of the wound care product that will be contacting the wound when the wound care product is applied to a wound. The upper layer of this embodiment, as depicted in Fig. 1 B, will face the opposite side and will thus initially not contact the wound when applied to the wound in this orientation. In the embodiment depicted in Fig. 1B, the upper layer may be considered a nanocellulose layer (1) and the lower layer may be considered a hydrogel layer (2) that has been applied to the nanocellulose layer.
While the invention has been illustrated and described in detail in the drawings and the foregoing description, such illustration and description are to be considered illustrative or exemplary and not restrictive.
From reading the present disclosure, other modifications will be apparent to persons skilled in the art. Such modifications may involve other features which are already known in the art and which may be used instead of or in addition to features already described herein.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality of elements or steps. The mere fact that certain measures are recited in mutually different dependent claims does not indicate that a combination of these measures cannot be used to advantage.

Any reference signs in the claims should not be construed as limiting the scope thereof.

**Definitions**

The term "decellularized ECM" as used herein refers to an ECM tissue from which a substantial amount of cellular and nucleic acid content has been removed leaving behind a complex interstitial structure of ECM that can be used as a scaffold for wound healing or tissue regeneration. In a preferred embodiment said term refers to an ECM material that has been cleaned, disinfected, sterilized and optionally cross-linked.

The term "hydrogel" refers to a three-dimensional (3D) crosslinked network of hydrophilic polymers that swell in water. In some embodiments, water can penetrate in between the polymer chains of the polymer network, subsequently causing swelling and the formation of a hydrogel. In general, hydrogels are superabsorbent. For example, in some embodiments, hydrogels can contain 50% or more, 60% or more, 70% or more, 80% or more, 90% or more, 95% or more, or 98% or more by weight of water. In some embodiments, further components such as proteins can be encapsulated within hydrogels. Typically, these further components are encapsulated within hydrogels through mixing a cell suspension and/or ECM molecules with a precursor solution (i.e., a solution comprising a polymer suitable for hydrogel formation) and crosslinking the resulting network using any available means for crosslinking. In some embodiments, a plurality of hydrogels can be assembled together to form a hydrogel assembly.

**List of reference numerals:**

1     Nanocellulose layer
2     Hydrogel

**Claims**

1. A multilayered wound care product comprising:

   (a) a layer comprising nanocellulose;
   (b) a hydrogel layer comprising at least one ECM-mimicking structure which is selected from the group consisting of particulate ECM, collagen protein structure, collagen-like protein structure and peptides comprising a matrix metalloproteinase (MMP) cleavage site, or a combination thereof.

2. The multilayered wound care product according claim 1, wherein the nanocellulose layer comprises a nanocellulose selected from the group consisting of cellulose nanofibers (CNF), nanocrystalline cellulose (NCC) and biocellulose produced by bacteria (BNC).

3. The multilayered wound care product according claim 1 or 2, wherein the BNC is produced by a microorganism of the group consisting of Acetobacter xylinum, Sphaerotilus spp., Pseudomonas spp., Alcaligenes spp., Salmonella spp., Agrobacterium spp., Rhizobium spp. and cyanobacterium expressing the exogenous acsAB genes from the cellulose synthase operon, and combinations thereof.

4. The multilayered wound care product according claims 1 to 3, wherein the hydrogel layer comprises an isolated polymer selected from the group consisting of chitosan, polyglycolic acid (PGA), polylactic acid (PLA) and polyethylene glycol.

5. The multilayered wound care product according to claim 4, wherein the polyethylene glycol is a linear, star, comb or preferably a branched PEG and even more preferably a branched PEG with four arms.

6. Wound dressing according to any of the above claims, wherein the collagen-like protein structure comprises the formula:

$$[(Gly\text{-}Xaa\text{-}Yaa)_m\text{-}(insert)_n]_p$$

where m is between 1 to 200 and $(Gly\text{-}Xaa\text{-}Yaa)_m$ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site.

7. The multilayered wound care product according to any of the above claims, wherein the peptide comprising a MMP cleavage site or the collagen like protein structure comprises one or more of the following:

a. a heparin binding dipeptide motif (BA)$_n$, wherein B is an amino acid with a positively charged side chain, A is alanine an n is an integer between 4 and 20;

b. a pH-labile chemical linker which is preferably to the C-terminally or N-terminally attached to said peptide;

c. an integrin binding site, being preferably an RGD motif or having an amino acid sequence of GFPGER (SEQ ID No. 26) or GEFYFDLRLK (SEQ ID NO. 27);

d. a photo-cleavable linker which is preferably to the C-terminally or N-terminally attached to said peptide;

e. an IKVAV (SEQ ID NO. 28) motif;

f. a fibronectin binding domain, having preferably the amino acid sequence of GLAGQRGIVGLPGQRGER (SEQ ID No. 29);

g. a Discoidin domain-containing receptor 2 (DDR2) domain, having preferably an amino acid sequence of GPRGQPGVMGFP (SEQ ID No. 30);

h. a non-collagen-natural break having a peptide sequence spaced between two glycine residues of the insert sequence of the collagen-like protein structure; having an amino acid sequence of SEQ ID No. 31 to 37;

i. a heparin binding domain;

j. an $\alpha 1\beta$-integrin binding domain, being preferably GLPGER, GFPGER or GFPGEN (SEQ ID Nos. 38-40)

k. an ECM retaining moiety comprising the amino acid sequence DHLSDNYTLDHDRAIH (SEQ ID No. 41);

l. an enzyme-sensitive peptide sequence, preferably being GPQGLLGAPGILGLP, GPQG↓IAGQ, GPQG↓IWGQ, GNVG↓LAGA, GPQG↓IA, GPQG↓IL, GPQG↓LA, GPQG↓LL, GPLG↓IA, GPLG↓IL, GPLG↓LA, GPLG↓LL, GPRG↓LQ, GPTG↓LA, GPQGIAGQRGVVGLP, GPQGLLGAPGILGLP, GEQGPQGLP, GPQGLAGQRGIV, GAQGPPGAPGPLGIAGITGARGLAGPPGMPGPRGS, GPLGIAGITGAR, GAQGPP-GAPGPLGIAGITGARGLA, GPSGAEGPPGPQGLAGQRGIVGLPGQRGERGFP, GPQGLAGQRGIV, GPS-GAEGPPGPQGLAGQRGIVGLP (SEQ ID Nos: 3-25), or GGGPQGIWGQGK

and whereby said peptide or collage-like protein structure is preferentially bound in a covalent or non-covalent form to the polymer and/or highly negative oligo-or polysaccharide of the hydrogel.

8. The multilayered wound care product according to any of the above claims, wherein the hydrogel of the hydrogel layer further comprises a highly negative oligo- or polysaccharide, whereby the hydrogel is preferably construed as an oligosaccharide/peptide/polymer system.

9. The multilayered wound care product according to claim 8, wherein the highly negative oligo- or polysaccharide is selected from the group consisting of heparin, dextran sulfate, $\alpha$-cyclodextrin sulfate, $\beta$-cyclodextrin sulfate, $\gamma$-cyclodextrin sulfate, $\alpha$-cyclodextrin phosphate, $\beta$-cyclodextrin phosphate, and $\gamma$-cyclodextrin phosphate.

10. The multilayered wound care product according to claim 9, wherein the highly negative oligosaccharide is dextran sulfate with a molecular weight between 4 to 600 kDa.

11. The multilayered wound care product according to any of the above claims, wherein the hydrogel layer comprises at least one cytokine, growth factor, hormone, or ECM-derived protein or any combination thereof.

12. The multilayered wound care product according to any of the above claims, wherein the hydrogel layer has a modulus of elasticity of at least 10 Pa.

13. The multilayered wound care product according to any of the above claims, wherein the hydrogel is a self-organizing hydrogel matrix.

14. The multi-layered wound care product according to any of the above claims, wherein the hydrogel layer adheres to the skin.

15. The multi-layered wound care product according to any of the above claims for use in the treatment of acute and chronic wounds, comprising burns, partial and full-thickness wounds, pressure ulcers, venous ulcers, arterial ulcers, diabetic ulcers, chronic vascular ulcers, draining wounds, tunnelled or undermined wounds, surgical wounds such as donor sites/grafts, post-Mohs surgery, post-laser surgery, podiatric dehiscence, and wound dehiscence, trauma wounds such as abrasions, lacerations, first-, second- or third degree burns, and skin tears.

16. A method of preparing a multilayered wound care product according to any of claims 1 to 15 comprising the steps of:

a. Producing the bio-nanocellulose:

i. cultivation of bacteria recombinantly expressing and excreting biocellulose
ii. depyrogenisation of the produced biocellulose
iii. washing- and neutralizing steps
iv. drying to adjust the water content of the product (mechanical, freeze-drying or the like)

b. Producing the hydrogel layer:

i. Optional: functionalizing the hydrogel monomer units with (bio)-functional domains
ii. Optional: addition of an aqueous solution of particulate ECM
iii. Preparation of the hydrogel and crosslinking of the monomer units
iv. washing steps

c. Producing the final wound care product

i. The product as resulting from step a) and the product as resulting from step b) are combined to the final wound care product.

**Fig. 1**

A

Star-PEG   Heparin

B

Europäisches
Patentamt

European
Patent Office

Office européen
des brevets

# EUROPEAN SEARCH REPORT

Application Number

EP 15 20 1164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/053960 A1 (BROWN R M JR [US] ET AL) 8 March 2007 (2007-03-08)<br>* paragraph [0037] - paragraph [0048] *<br>* paragraph [0055] *<br>* paragraph [0061] - paragraph [0063] *<br>* paragraph [0066] - paragraph [0085]; claims; examples * | 1-16 | INV.<br>A61L15/24<br>A61L15/32<br>A61L15/58<br>C08L1/26<br>C07K14/78<br>A61L15/26<br>C08L71/02 |
| Y | MIKHAIL V. TSURKAN ET AL: "Modular StarPEG-Heparin Gels with Bifunctional Peptide Linkers",<br>MACROMOLECULAR RAPID COMMUNICATIONS,<br>vol. 31, no. 17,<br>16 August 2010 (2010-08-16), pages 1529-1533, XP055102320,<br>ISSN: 1022-1336, DOI: 10.1002/marc.201000155<br>* the whole document * | 1-16 | |
| A | WO 98/00180 A1 (JOHNSON & JOHNSON MEDICAL [US]; WATT PAUL WILLIAM [GB]; HARVEY WILSON) 8 January 1998 (1998-01-08)<br>* page 1, lines 21-26 *<br>* page 7, lines 17-30 *<br>* page 11, paragraph 5 - line 11 *<br>* claims; examples * | 1-16 | TECHNICAL FIELDS SEARCHED (IPC)<br><br>A61L<br>C08L<br>C07K |
| A | US 2013/330417 A1 (DONG HONG [US] ET AL) 12 December 2013 (2013-12-12)<br>* paragraphs [0015], [0016], [0018] *<br>* paragraphs [0025] - [0033] * | 1-16 | |

-/--

The present search report has been drawn up for all claims

| Place of search | Date of completion of the search | Examiner |
|---|---|---|
| The Hague | 9 June 2016 | Derrien, Anne-Cécile |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 1 of 2

EP 3 181 152 A1

**EUROPEAN SEARCH REPORT**

Application Number

EP 15 20 1164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | G. SUN ET AL: "Dextran hydrogel scaffolds enhance angiogenic responses and promote complete skin regeneration during burn wound healing", PROCEEDINGS OF THE NATIONAL ACADEMY OF SCIENCES, vol. 108, no. 52, 27 December 2011 (2011-12-27), pages 20976-20981, XP55159511, ISSN: 0027-8424, DOI: 10.1073/pnas.1115973108 * the whole document * | 1-16 | |
| A | FREUDENBERG U ET AL: "A star-PEG-heparin hydrogel platform to aid cell replacement therapies for neurodegenerative diseases", BIOMATERIALS, ELSEVIER SCIENCE PUBLISHERS BV., BARKING, GB, vol. 30, no. 28, 1 October 2009 (2009-10-01), pages 5049-5060, XP026420872, ISSN: 0142-9612 [retrieved on 2009-06-27] * the whole document * | 1-16 | |

TECHNICAL FIELDS
SEARCHED (IPC)

The present search report has been drawn up for all claims

| Place of search | Date of completion of the search | Examiner |
|---|---|---|
| The Hague | 9 June 2016 | Derrien, Anne-Cécile |

page 2 of 2

## ANNEX TO THE EUROPEAN SEARCH REPORT
## ON EUROPEAN PATENT APPLICATION NO.

EP 15 20 1164

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2016

| Patent document cited in search report | | Publication date | Patent family member(s) | | Publication date |
|---|---|---|---|---|---|
| US 2007053960 | A1 | 08-03-2007 | US | 2007053960 A1 | 08-03-2007 |
| | | | WO | 2007027849 A2 | 08-03-2007 |
| WO 9800180 | A1 | 08-01-1998 | AT | 247493 T | 15-09-2003 |
| | | | AT | 362381 T | 15-06-2007 |
| | | | AU | 737809 B2 | 30-08-2001 |
| | | | BR | 9710177 A | 18-01-2000 |
| | | | CA | 2258990 A1 | 08-01-1998 |
| | | | CZ | 9804251 A3 | 12-05-1999 |
| | | | DE | 69724257 D1 | 25-09-2003 |
| | | | DE | 69724257 T2 | 01-07-2004 |
| | | | DE | 69737741 T2 | 31-01-2008 |
| | | | EP | 0918548 A1 | 02-06-1999 |
| | | | EP | 1325754 A1 | 09-07-2003 |
| | | | ES | 2205237 T3 | 01-05-2004 |
| | | | ES | 2287406 T3 | 16-12-2007 |
| | | | GB | 2314842 A | 14-01-1998 |
| | | | JP | 4401438 B2 | 20-01-2010 |
| | | | JP | 2000513258 A | 10-10-2000 |
| | | | KR | 20000022287 A | 25-04-2000 |
| | | | PL | 330824 A1 | 07-06-1999 |
| | | | PT | 918548 E | 28-11-2003 |
| | | | WO | 9800180 A1 | 08-01-1998 |
| US 2013330417 | A1 | 12-12-2013 | US | 2013330417 A1 | 12-12-2013 |
| | | | US | 2014213764 A1 | 31-07-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 2888331 A1
- EP 0186495 A
- US 4378431 A1
- US 5962648 A
- US 5593859 A
- WO 2004078120 A
- US 6953839 B
- WO 2010091251 A
- WO 2014040591 A

**Non-patent literature cited in the description**

- **KREBS et al.** *J Am. Chem. Soc.,* 2009, vol. 131 (26), 9204-9206
- **GILS et al.** *Am. J Biomed. Sei,* 2010, vol. 2 (4), 373-383
- **BALAKRISHNAN ; JAYAKRISHNAN.** *Biomaterials,* 2005, vol. 26 (18), 3941-3951
- **WANG et al.** *Pharmaceutical Res.,* 2011, vol. 28 (6), 1406-1414
- **FITTKAU et al.** *Biomaterials,* 2005, vol. 26, 167-174
- **JUNG et al.** *Appl Biochem Biotechnol,* 2010, vol. 162, 486-497
- **FREUDENBERG et al.** *Biomaterials,* 2009, vol. 30, 5049-5060
- **SUN et al.** *PNAS,* 2011, vol. 108 (52), 20976-20981
- **LIU et al.** *Macromol Rapid Comm,* 2010, vol. 31, 1148-1154
- **ALI et al.** *BioResearch Open Access,* 2013, vol. 2 (4), 241-249
- **MAITZ et al.** *Nature Communications,* 2013, vol. 4

SEQUENCE LISTING

<110> BSN Medical GmbH

<120> Multi-layered wound care product

<130> 14718-PT-EP

<160> 45

<170> BiSSAP 1.3.5

<210> 1
<211> 16
<212> PRT
<213> Artificial Sequence

<223> "Peptide for cross-linking hydrogel"

<220>
<223> Peptide for cross-linking hydrogel

<400> 1
Gly Cys Arg Asp Gly Asp Gln Gly Ile Ala Gly Phe Asp Arg Cys Gly
1               5                   10                  15


<210> 2
<211> 16
<212> PRT
<213> Artificial Sequence

<223> "Peptide for cross-linking hydrogel"

<220>
<223> Peptide for cross-linking hydrogel

<400> 2
Gly Cys Arg Asp Gly Asp Gln Gly Ile Ala Gly Phe Asp Arg Cys Gly
1               5                   10                  15


<210> 3
<211> 8
<212> PRT
<213> Artificial Sequence

<223> "Cleavage after Gly4"

<220>
<223> Cleavage after Gly4

<400> 3
Gly Pro Gln Gly Ile Ala Gly Gln
1               5

<210> 4
<211> 8
<212> PRT
<213> Artificial Sequence

```
<223> "Cleavage after Gly4"

<220>
<223> Cleavage after Gly4

<400> 4
Gly Pro Gln Gly Ile Trp Gly Gln
1               5

<210> 5
<211> 8
<212> PRT
<213> Artificial Sequence

<223> "Cleavage after Gly4"

<220>
<223> Cleavage after Gly4

<400> 5
Gly Asn Val Gly Leu Ala Gly Ala
1               5

<210> 6
<211> 6
<212> PRT
<213> Artificial Sequence

<223> "Cleavage after Gly4"

<220>
<223> Cleavage after Gly4

<400> 6
Gly Pro Gln Gly Ile Ala
1               5

<210> 7
<211> 6
<212> PRT
<213> Artificial Sequence

<223> "Cleavage after Gly4"

<220>
<223> Cleavage after Gly4

<400> 7
Gly Pro Gln Gly Ile Leu
1               5

<210> 8
<211> 6
<212> PRT
<213> Artificial Sequence

<223> "Cleavage after Gly4"

<220>
<223> Cleavage after Gly4

<400> 8
Gly Pro Gln Gly Leu Ala
```

```
<210> 9
<211> 6
<212> PRT
<213> Artificial Sequence

<223> "Cleavage after Gly4"

<220>
<223> Cleavage after Gly4

<400> 9
Gly Pro Gln Gly Leu Leu
1               5


<210> 10
<211> 6
<212> PRT
<213> Artificial Sequence

<223> "Cleavage after Gly4"

<220>
<223> Cleavage after Gly4

<400> 10
Gly Pro Leu Gly Ile Ala
1               5


<210> 11
<211> 6
<212> PRT
<213> Artificial Sequence

<223> "Cleavage after Gly4"

<220>
<223> Cleavage after Gly4

<400> 11
Gly Pro Leu Gly Ile Leu
1               5


<210> 12
<211> 6
<212> PRT
<213> Artificial Sequence

<223> "Cleavage after Gly4"

<220>
<223> Cleavage after Gly4

<400> 12
Gly Pro Leu Gly Leu Ala
1               5


<210> 13
<211> 6
<212> PRT
<213> Artificial Sequence
```

```
<223> "Cleveage after Gly4"

<220>
<223> Cleveage after Gly4

<400> 13
Gly Pro Leu Gly Leu Leu
1               5

<210> 14
<211> 6
<212> PRT
<213> Artificial Sequence

<223> "Cleavage after Gly4"

<220>
<223> Cleavage after Gly4

<400> 14
Gly Pro Arg Gly Leu Gln
1               5

<210> 15
<211> 6
<212> PRT
<213> Artificial Sequence

<223> "Cleavage after Gly4"

<220>
<223> Cleavage after Gly4

<400> 15
Gly Pro Thr Gly Leu Ala
1               5

<210> 16
<211> 15
<212> PRT
<213> Homo sapiens

<223> "Collagen fragment"

<220>
<223> Collagen fragment

<400> 16
Gly Pro Gln Gly Ile Ala Gly Gln Arg Gly Val Val Gly Leu Pro
1               5                       10                      15

<210> 17
<211> 15
<212> PRT
<213> Homo sapiens

<223> "collagen fragment"

<220>
<223> collagen fragment

<400> 17
Gly Pro Gln Gly Leu Leu Gly Ala Pro Gly Ile Leu Gly Leu Pro
```

27

1                    5                          10                              15

```
<210> 18
<211> 9
<212> PRT
<213> Homo sapiens

<223> "repeat tripeptide sequence"

<220>
<223> repeat tripeptide sequence

<400> 18
Gly Glu Gln Gly Pro Gln Gly Leu Pro
1               5

<210> 19
<211> 12
<212> PRT
<213> Homo sapiens

<223> "repeat tripeptide sequence"

<220>
<223> repeat tripeptide sequence

<400> 19
Gly Pro Gln Gly Leu Ala Gly Gln Arg Gly Ile Val
1               5                       10

<210> 20
<211> 35
<212> PRT
<213> Homo sapiens

<223> "repeat tripeptide sequence"

<220>
<223> repeat tripeptide sequence

<400> 20
Gly Ala Gln Gly Pro Pro Gly Ala Pro Gly Pro Leu Gly Ile Ala Gly
1               5                   10                      15
Ile Thr Gly Ala Arg Gly Leu Ala Gly Pro Pro Gly Met Pro Gly Pro
            20                      25                      30
Arg Gly Ser
            35

<210> 21
<211> 12
<212> PRT
<213> Homo sapiens

<223> "repeat tripeptide sequence"

<220>
<223> repeat tripeptide sequence

<400> 21
Gly Pro Leu Gly Ile Ala Gly Ile Thr Gly Ala Arg
1               5                   10

<210> 22
```

```
<211> 24
<212> PRT
<213> Homo sapiens


<400> 22
Gly Ala Gln Gly Pro Pro Gly Ala Pro Gly Pro Leu Gly Ile Ala Gly
1               5                   10                  15
Ile Thr Gly Ala Arg Gly Leu Ala
                20


<210> 23
<211> 33
<212> PRT
<213> Homo sapiens

<223> "repeat tripeptide sequence"

<220>
<223> repeat tripeptide sequence

<400> 23
Gly Pro Ser Gly Ala Glu Gly Pro Pro Gly Pro Gln Gly Leu Ala Gly
1               5                   10                  15
Gln Arg Gly Ile Val Gly Leu Pro Gly Gln Arg Gly Glu Arg Gly Phe
                20                  25                  30
Pro


<210> 24
<211> 12
<212> PRT
<213> Homo sapiens

<223> "repeat tripeptide sequence"

<220>
<223> repeat tripeptide sequence

<400> 24
Gly Pro Gln Gly Leu Ala Gly Gln Arg Gly Ile Val
1               5                   10


<210> 25
<211> 24
<212> PRT
<213> Homo sapiens

<223> "repeat tripeptide sequence"

<220>
<223> repeat tripeptide sequence

<400> 25
Gly Pro Ser Gly Ala Glu Gly Pro Pro Gly Pro Gln Gly Leu Ala Gly
1               5                   10                  15
Gln Arg Gly Ile Val Gly Leu Pro
                20


<210> 26
<211> 6
<212> PRT
<213> Homo sapiens
```

<223> " 2 1 integrin binding site"

<220>
<223>  2 1 integrin binding site

<400> 26
Gly Phe Pro Gly Glu Arg
1               5

<210> 27
<211> 10
<212> PRT
<213> Homo sapiens

<223> " 2 1 integrin binding site"

<220>
<223>  2 1 integrin binding site

<400> 27
Gly Glu Phe Tyr Phe Asp Leu Arg Leu Lys
1               5                   10

<210> 28
<211> 5
<212> PRT
<213> Mus musculus

<223> "laminin alpha1 chain peptide"

<220>
<223> laminin alpha1 chain peptide

<400> 28
Ile Lys Val Ala Val
1               5

<210> 29
<211> 18
<212> PRT
<213> Homo sapiens

<223> "collagen derived sequence"

<220>
<223> collagen derived sequence

<400> 29
Gly Leu Ala Gly Gln Arg Gly Ile Val Gly Leu Pro Gly Gln Arg Gly
1               5                   10                  15
Glu Arg


<210> 30
<211> 12
<212> PRT
<213> Homo sapiens

<223> "Discoidin domain-containing receptor 2 (DDR2) domain"

<220>
<223> Discoidin domain-containing receptor 2 (DDR2) domain

```
<400> 30
Gly Pro Arg Gly Gln Pro Gly Val Met Gly Phe Pro
1               5                   10

<210> 31
<211> 6
<212> PRT
<213> Homo sapiens

<223> " from  5 chain of type IV collagen"

<220>
<223> from  5 chain of type IV collagen

<400> 31
Gly Ala Ala Val Met Gly
1               5

<210> 32
<211> 8
<212> PRT
<213> Homo sapiens

<223> "from  1 (VII) collagen"

<220>
<223> from  1 (VII) collagen

<400> 32
Gly Asp Ser Ala Val Ile Leu Gly
1               5

<210> 33
<211> 10
<212> PRT
<213> Homo sapiens

<223> "from  4 (IV) collagen"

<220>
<223> from  4 (IV) collagen

<400> 33
Gly Asp Met Val Val Ser Arg Val Lys Gly
1               5                   10

<210> 34
<211> 14
<212> PRT
<213> Homo sapiens

<223> "from  1 (VII) collagen"

<220>
<223> from  1 (VII) collagen

<400> 34
Gly Arg Leu Val Asp Thr Gly Pro Gly Ala Arg Glu Lys Gly
1               5                   10

<210> 35
<211> 43
```

<212> PRT
<213> Homo sapiens

<223> "from  1 (VII) collagen"

<220>
<223> from  1 (VII) collagen

<400> 35
Gly Ser Val Pro Asn Val Asp Arg Leu Leu Glu Thr Ala Gly Ile Lys
1               5                   10                  15
Ala Ser Ala Leu Arg Glu Ile Val Glu Thr Trp Asp Glu Ser Ser Gly
            20                  25                  30
Ser Phe Leu Pro Val Pro Glu Arg Arg Arg Gly
        35                  40

<210> 36
<211> 15
<212> PRT
<213> Homo sapiens

<400> 36
Gly Pro Gly Glu Phe Tyr Phe Asp Leu Arg Leu Lys Gly Pro Gly
1               5                   10                  15

<210> 37
<211> 16
<212> PRT
<213> Homo sapiens

<400> 37
Gly Gln Ile Ser Glu Gln Lys Arg Pro Ile Asp Val Glu Phe Gln Lys
1               5                   10                  15

<210> 38
<211> 6
<212> PRT
<213> Homo sapiens

<400> 38
Gly Leu Pro Gly Glu Arg
1               5

<210> 39
<211> 6
<212> PRT
<213> Homo sapiens

<400> 39
Gly Phe Pro Gly Glu Arg
1               5

<210> 40
<211> 6
<212> PRT
<213> Homo sapiens

<400> 40
Gly Phe Pro Gly Glu Asn
1               5

<210> 41
<211> 16
<212> PRT
<213> Homo sapiens

<400> 41
Asp His Leu Ser Asp Asn Tyr Thr Leu Asp His Asp Arg Ala Ile His
1               5                   10              15

<210> 42
<211> 67
<212> PRT
<213> Streptococcus pyogenes

<400> 42
Glu Glu Asn Glu Lys Val Arg Glu Gln Glu Lys Leu Ile Gln Gln Leu
1               5                   10              15
Ser Glu Lys Leu Val Glu Ile Asn Asp Leu Gln Thr Leu Asn Gly Asp
            20                  25                  30
Lys Glu Ser Ile Gln Ser Leu Val Asp Tyr Leu Thr Arg Arg Gly Lys
            35                  40                  45
Leu Glu Glu Glu Trp Met Glu Tyr Leu Asn Ser Gly Ile Gln Arg Lys
        50                  55                  60
Leu Phe Val
65

<210> 43
<211> 86
<212> PRT
<213> Rhodopseudomonas palustris

<400> 43
Pro Ser Ile Glu Gln Val Met Pro Trp Leu His Leu Ile Phe Asp Ala
1               5                   10              15
Tyr Glu Asp Tyr Lys Ala Gln Arg Ala Arg Glu Ala Arg Glu Leu Glu
            20                  25                  30
Glu Arg Leu Ala Ala Glu Ala Leu Glu Gln Ala Ala Arg Glu Ala Ala
        35                  40                  45
Glu Arg Glu Val Ala Ala Ala Ile Glu Ala Ala Asn Ala Glu Ala Glu
        50                  55                  60
Ile Met Leu Asp Asp Glu Thr His Ala Glu Gly Gly Lys Lys Lys Lys
65                  70                  75                  80
Lys Arg Lys His Lys Asp
                85

<210> 44
<211> 11
<212> PRT
<213> Artificial Sequence

<223> "integrin binding peptide"

```
<220>
<223> Integrin binding peptide

<400> 11
Gly Cys Gly Gly Gly Arg Gly Asp Ser Pro Gly
1               5                   10


<210> 45
<211> 12
<212> PRT
<213> Artificial Sequence

<223> "protease-sensitive peptide"

<220>
<223> Protease-sensitive peptide

<400> 12
Gly Gly Gly Pro Gln Gly Ile Trp Gly Gln Gly Lys
1               5                   10
```